(19) Europäisches Patentamt
European Patent Office
Office européen des brevets

(11) **EP 2 253 308 A1**

(12) # EUROPEAN PATENT APPLICATION

(43) Date of publication:
**24.11.2010 Bulletin 2010/47**

(51) Int Cl.:
**A61K 9/127** (2006.01)   **A61K 49/22** (2006.01)
**A61K 31/704** (2006.01)   **A61K 9/133** (2006.01)

(21) Application number: **09160913.1**

(22) Date of filing: **22.05.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicants:
• **Ludwig-Maximilians-Universität München**
  **80359 München (DE)**
• **Ruprecht-Karls-Universität Heidelberg**
  **69117 Heidelberg (DE)**

(72) Inventors:
• **Winter, Gerhard**
  **82377 Penzberg (DE)**

• **Coester, Conrad**
  **36119 Neuhof (DE)**
• **Tinkov, Stelijan**
  **81375 München (DE)**
• **Bekeredjian, Raffi**
  **69251 Gaiberg (DE)**

(74) Representative: **Neuefeind, Regina**
  **Maiwald Patentanwalts GmbH**
  **Elisenhof**
  **Elisenstrasse 3**
  **80335 München (DE)**

(54) **Pharmaceutical composition comprising microbubbles for targeted tumor therapy**

(57) The present invention relates to a pharmaceutical composition for the targeted delivery of a pharmaceutically active compound, the composition comprising soft, gas-filled particles having an envelope monolayer comprising one or more surfactants and a pharmaceutically active compound. More particularly, the invention relates to a pharmaceutical composition, wherein the envelope monolayer of the gas-filled particles have a negative overall net charge, and wherein the pharmaceutically active compound has a positive overall net charge and is directly bound to one or more surfactants by both electrostatic and hydrophobic interactions. The present invention also relates to a method of producing said pharmaceutical composition comprising the steps of providing a pharmaceutical active compound- loaded liposomal preparation and the subsequent formation of gas-filled particles by adding a pharmaceutically acceptable gas to the liposomal preparation. Furthermore, the invention relates to the use of said pharmaceutical composition in the treatment of tumors.

EP 2 253 308 A1

**Description**

**FIELD OF THE INVENTION**

The present invention relates to a pharmaceutical composition for the targeted delivery of a pharmaceutically active compound, the composition comprising soft, gas-filled particles having an envelope monolayer comprising one or more surfactants and a pharmaceutically active compound. More particularly, the invention relates to a pharmaceutical composition, wherein the envelope monolayer of the gas-filled particles have a negative overall net charge, and wherein the pharmaceutically active compound has a positive overall net charge and is directly bound to one or more surfactants by both electrostatic and hydrophobic interactions. The present invention also relates to a method of producing said pharmaceutical composition comprising the steps of providing a pharmaceutical active compound-loaded liposomal preparation and the subsequent formation of gas-filled particles by adding a pharmaceutically acceptable gas to the liposomal preparation. Furthermore, the invention relates to the use of said pharmaceutical composition in the treatment of tumors.

**BACKGROUND**

The use of medications to treat cancer has played a major role for over half a century. It still remains a challenging task to treat solid tumors locally and in a targeted way. Many chemotherapeutic agents have been extensively tested and evaluated with regard to their therapeutic potential to effectively destroy cancer cells. For example, anthracycline antibiotics are commonly used as chemotherapeutic agents. One of the most potent anthracycline antibiotic is doxorubicin, which is used in the treatment of a wide range of cancers, specifically haematological malignancies, acute lymphoblastic leukemia, acute myeloblastic leukemia, neuroblastoma, many types of carcinomas, and soft tissue and bone sarcomas. The drug is administered intravenously in the form of a solution of its hydrochloride salt (Adriamycin RDF®, Pharmacia, Inc., USA). Doxorubicin is known to interact with DNA by intercalation between the nucleotide pairs, which in turn leads to inhibition of the DNA topoisomerase II enzyme, thus blocking the process of DNA replication in target cells. Doxorubicin has also been reported as being highly cytotoxic without entering the cells due to its ability to interact with biological membranes and phospholipids. Specifically, this could lead to alterations in membrane fluidity, transport characteristics, reorganization of membrane lipid domains and inhibition of several cardiolipin-dependent respiratory chain processes in mitochondria. Interference of doxorubicin with mitochondrial activity is generally thought to contribute to cardiotoxicity.
However, the conventional application of the free chemotherapeutic agent for the treatment of solid tumors has encountered several severe drawbacks in clinical practice. This is because chemotherapeutic agents can also affect healthy cells of the body, resulting in severe side effects such as nausea, vomiting, immunotoxicity, cardiotoxicty, liver damage, etc. In order to circumvent these disadvantages it is a major task in medicinal and pharmaceutical development to directly and specifically deliver a chemotherapeutic agent to the tumor locus, thereby minimizing the burden on other tissues and organs. In contrast to conventional application of the free drug, targeted delivery thus can attenuate the side effects and interference away from the target site. Furthermore, the effective dosage of an agent can be considerably reduced to improve the overall therapeutic efficacy.
The use of nanometer-sized particles as carriers for bioactive agents, in particular chemotherapeutics, is known in the art. The encapsulation in micelles or other nano-particles offers the advantage of being able to decrease the systemic concentration of free drug and therefore to diminish the intracellular uptake by normal healthy cells. One further benefit of nanometer-scaled carriers is their enhanced ability to penetrate tumor tissues through abnormally leaky capillary vasculature and subsequently to be entrapped *via* the enhanced permeability and retention (EPR) effect. It is also known that an effective drug uptake by tumor cells can be triggered by locally irradiating with therapeutic ultrasound (US). The irradiation then results in an increased drug influx, which could help to overcome drug resistance caused by the action of efflux pumps [Rapoport, N., Controlled Drug Delivery to Drug Sensitive and Multidrug Resistant Cells: Effects of Pluronic Micelles and Ultrasound in: Advances in Controlled Drug Delivery. ACS Symposium Book Series, ed S. Dinh and P. Liu, Washington DC, 2003, pp. 85-101].
For instance, targeted delivery of chemotherapeutical agents to tumors has been approached by making use of liposomes as carriers. Liposomes, also known as lipid vesicles, are completely closed lipid bilayer membranes which contain an entrapped volume comprising an aqueous medium. The lipid bilayer is often composed of phospholipids such as lecithin and related materials such as glycolipids. The bilayer is composed of two lipid mono layers, each having a hydrophobic portion oriented towards each other with the hydrophilic portion facing outwards towards the aqueous phase. Liposomes bearing doxorubicin in their aqueous core are well established and already applied in clinical practice. Marketed products are, for example, Doxil® (Ortho Viotech, USA), Myocet® (Cephalon, EU) and Caelyx® (Shering Plough, USA).
Anthracyclines anchored to liposomes through a chemically modified palmitoyl residue leading to the incorporation of the drug into the phospholipid bilayer of liposomes are also described in the art [Perez-Soler, R., Priebe, W.,

Liposomal formulation and antitumor activity of 14-O-palmitoyl-hydroxyrubicin. Cancer Chemother Pharmacol, 1992. 30: 267-271].

A further advance in the art was the decoration of liposomes with targeting ligands such as monoclonal antibodies. These so called "immunoliposomes" have the ability to specifically bind to receptors, which are only overexpressed in tumors, thus leading to the accumulation of the bioactive agent at the site of interest [Siwak, D., Tary, A.M., Lopes-Berestein, G., The potential of drug-carrying immunoliposomes as anticancer agents. Clin Canc Res, 2002. 8: 955-956.].

Several other approaches make full use of the positive charge and the amphiphilic properties of the doxorubicin molecule for its attachment to anionic charged liposomes. Formulations of doxorubicin-containing liposomes comprising anionic phospholipids are well known in the art [Amselem, S. et al., Optimization and upscaling of doxorubicin-containing liposomes for clinical use. J Pharm Scie, 1990. 79 (12): 1045.1052; WO 88/09168, US 4,898,735, US 4,797,285).

In recent years, several approaches have been developed which have attempted to combine the qualities of ultrasound imaging and targeted tumor therapy. Specifically, these techniques are based on micron- and submicron-sized, gas-filled particles, often called microbubbles (MBs) which have proven to be suitable carriers for targeted gene and drug delivery. The use of microbubble-containing suspensions as efficient ultrasound reflectors is known in the art. This is due to their significant difference in acoustic impedance relative to blood, which renders these gas-filled particles an excellent contrast agent for ultrasound imaging and diagnostics. Today, injectable formulations comprising acoustically active particles are commonly used as ultrasound contrast agents in diagnostic practice. Generally, these suspensions include microbubbles having a diameter of a few microns dispersed in an aqueous medium. Microbubbles are gas-filled colloidal particles of 1 to 8 $\mu$m in size consisting of a core of gas, which is encapsulated in a more or less flexible shell of protein, surfactant, or polymer. The gas core can be either a poorly water-soluble gas or a volatile liquid (gas precursor), which can spontaneously undergo a liquid-to-gas shift upon temperature increase, pressure drop, or exposure to ultrasound.

The development of the first microbubble-based ultrasonic contrast agent has brought great improvement into ultrasound diagnostics. Initially these contrast agents were mainly used for right heart opacification and cardiac shunt diagnostics. The development of smaller gas-filled microbubbles allowed systemic circulation of an effective ultrasound contrast agent after intravenous injection. For example, the marketed product SonoVue® (Bracco, USA) is a second-generation ultrasound contrast agent comprising microbubbles whose shell consists of phosphoplids. The gas core consists of sulphur hexafluoride ($SF_6$), which is barely soluble in blood and is physiologically inert. This improvement resulted in MBs with a prolonged half-life.

In the past years ultrasound-targeted microbubble destruction (UTMD) has evolved as a new and promising tool for site-specific gene and drug delivery. One major advantage of acoustically active microbubbles compared to other above mentioned drug carriers such as liposomes, is that they can be easily visualized and precisely destroyed through diagnostic ultrasound, thereby releasing their therapeutic payload at the target site. After intravenous injection, microbubbles will opacify blood-filled cavities and increase tissue contrast intensities of well perfused organs. MBs subjected to US of their resonance frequency will oscillate depending on the US energy. At low mechanical index (MI) microbubbles remain stable despite their oscillations. At high mechanical index ultrasound, however, the high-amplitude oscillations lead to fragmentation of microbubbles. Microbubbles which are loaded with a pharmaceutically active agent will thus release their therapeutic payload upon fragmentation into their close proximity. The fragmentation of microbubbles in the ultrasound field is generally dependent on the physico-chemical properties of the MB shell, MB diameter and the amount and qualitative nature of the substance delivered. A further advantage is that the local application of ultrasound can transiently enhance permeability of several biological barriers, such as the blood-brain barrier, small blood vessels, or cell membranes, thereby facilitating the delivery and uptake of pharmaceutically active agents into tissues and cells.

Depending on their structure, acoustically active particles can be generally classified as follows:

1) Rigid shelled microbubbles and microcapsules having shells consisting of serum albumin or a synthetic polymer, and cores of gas
2) Perfluorocarbon droplets having shells consisting of surfactants and cores of a gaseous precursor
3) Acoustically active lipospheres having an outer phospholipid monolayer, an underlying oil layer, and a gas core
4) Soft-shelled microbubbles having shells of surfactants and cores of gas.

For instance, formulations of doxorubicin-loaded microcapsules with rigid polymeric shells have been described by Burstein *et al.* [Burstein, O., Wheatley, M.A., Drug loaded contrast agents for cancer tumors: A combination of imaging and therapy. Biomedical Technology Showcase, School of Biomedical Engineering, Science& health Systems, Drexel University, PA, 2006.], and in WO 2007/137326. Specifically, these echogenic microcapsules are based on either Poly-lactic acid (PLA) or Poly-lactic-co-glycolic acid (PLGA) used as the shell polymer. These microbubbles can be filled with any biocompatible gas.

WO 2005/030171 relates to pharmaceutical compositions comprising rigid-shelled microbubbles which contain a gas selected from a perfluorocarbon and $SF_6$ encapsulated with a filmogenic protein such as serum albumin for the delivery of an antiproliferative therapeutic agent. In particular, the prior art describes microbubbles comprising a dextrose/ albumin shell and a perfluorocarbon core, also termed PESDA (perfluorocarbon-exposed sonicated dextrose/albumin). These microbubbles are non-covalently conjugated to the therapeutic agent such that it binds at the gas/fluid interface of the microbubbles.

The ultrasound contrast agent Echogen® (Sonus Pharmaceuticals Ltd., USA) (which has meanwhile been withdrawn from the US market) is based on a surfactant- stabilized microemulsion of perfluoropentane in an aqueous medium.

WO 2006/127953 further developed this concept towards the targeted delivery of chemotherapeutic agents. In particular, this document describes compositions, wherein drug-loaded microbubbles are mixed with bioactive agents encapsulated in polymeric micelles or other nano-particles. Polymeric micelles are nano-sized efficient carriers of a hydrophobic drug, which are encapsulated in the inner core of the micelle. The microbubbles are produced *in situ* upon injection of a perfluoropentane microemulsion and are stabilized by shells consisting of a synthetic block copolymer. Upon activation through temperature increase, the injected perfluoropentane microemulsion undergoes a phase shift, and spontaneously forms a mixture of drug-loaded micelles, droplets, and nanometer-sized microbubbles. The perfluoropentane-containing microbubbles having polymeric shells require the application of high intensity focused ultrasound (HIFU) in order to be destroyed at the site of tumor.

US 2002/0159952 describes Paclitaxel-loaded acoustically active lipospheres (AALs). In particular, the disclosure is directed to a targeted therapeutic delivery system comprising gas-filled microspheres having a shell consisting of a surfactant monolayer, an underlying oil layer, e.g. triacetin, and a therapeutic agent, e.g. Paclitaxel, which is dissolved therein.

Further advances in the field have led to the development of pharmaceutical preparations wherein the microbubbles are indirectly associated with the therapeutic agent *via* a second vesicular component. For instance, WO 1999/39738 discloses formulations comprising liposomes filled with a bioactive substance and gas-filled microbubbles associated thereto. The liposomes are bound to the microbubbles through the linking interaction of complementary moieties such as biotin and avidin.

More recently, US 2007/081946 describes compositions comprising gas-filled microbubbles capable of binding to so called microvesicle associated components (MACs) through electrostatic interaction. Said MACs can be any supramolecular structure such as a micelle, a colloidal nano-particle, or a solid polymeric nano-particle which harbours a targeting ligand or a bioactive agent in the interior.

However, the above described microbubble and nano-particle formulations of the prior art have several significant disadvantages. The therapeutic value of these formulations, especially with regard to targeted delivery, primarily depends on both, the echogenicity (backscatter activity) and destructibility of the gas-filled nano-particles. The acoustic properties, in turn, are mainly dependent on the acoustic impedance of the core component in conjunction with the flexibility of the shell. For instance, perfluorocarbon droplets as described in WO 2006/127953 have a rather low US backscatter activity because their acoustic impedance is closer to water than that of gases. Rigid shelled microbubbles and microcapsules, for example, having envelopes made of block copolymers or filmogenic proteins produce less intensive and shorter contrast in the ultrasonic field. These particles therefore require considerably higher ultrasound energy doses in order to be destroyed at the site of tumor. However, the application of high intensity ultrasound (HIFU) in tumors is highly problematic because it can be accompanied by undesirable and unpredictable effects such as acoustic damage or potential promotion of tumor metastasizing.

The same is true for acoustically active lipospheres (AALs) as described in US 2002/0159952. The use of an additional underlying oil layer as the carrier of a pharmaceutical active compound has the basic disadvantage that echogenicity and destructibility of these microbubbles are decreased when US is applied.

A further problem is the structural complexity of some formulations having mixed vesicle populations, wherein at least two or more different vesicle types are involved. For example, the perfluoropentane droplets as described in WO 2006/127953 represent a mixture of *in situ* formed microbubbles and drug-containing polymeric micelles. On the one hand it remains difficult to control the process of *in situ* microbubble formation so that the application of US cannot be performed immediately after administration. On the other hand, due to the broad and multimodal size distribution, cavitation control and dosage of such complex formulations are impeded.

Further, the use of biocompatible and biodegradable excipients is an important consideration in clinical practice. It is still not clear whether the above mentioned substances such as perfluoropentane and block- or diblock-copolymers can be used without generating side effects.

Hence, there is a strong need in the field to provide an improved acoustically active drug delivery system for targeted tumor therapy. The problem of producing gas-filled particles which combine the qualities of an excellent carrier of a pharmaceutically active compound with the desired acoustic properties required in clinical practice still remains unsolved. In particular, microbubbles although loaded with a pharmaceutically active agent should have a sufficiently high backscatter efficacy allowing for tumor visualization and localization. Furthermore, there is an ongoing quest to provide microbubbles having a high acoustic destructibility which would allow the use of ultrasound at well-tolerated intensities. There is also a need to provide formulations having a narrow and homogenous size distribution of microbubbles. This is important since both the amount of loaded chemotherapeutic agent and the acoustic qualities are highly dependent on the particle size of the produced microbubbles. Finally, there is still an urgent need to provide compositions which are mainly based on pharmaceutically acceptable, non-irritating, and biocompatible excipients.

Accordingly, it is an object of the present invention to provide a novel and simple drug delivery system which meets the above mentioned criteria. The present invention provides a pharmaceutical composition comprising soft, gas-filled, acoustically active particles having an envelope monolayer of at least one or more surfactants and a pharmaceutically active agent. The pharmaceutically active agent to be delivered is directly bound to the one or more surfactants by both electrostatic and hydrophobic interactions. In particular, the soft gas-filled particles according to the invention have revealed an unexpectedly high drug-binding and a high drug targeting capacity. Surprisingly, the pharmaceutically active agent-loaded microbubbles of the present invention have demonstrated improved acoustic properties in both *in vitro* and *in vivo* experiments, rendering them excellent vehicles for targeted delivery in tumor therapy. The *in vivo* echogenicity and destructibility of these drug-loaded microbubbles was unexpectedly found to be greater than that of conventional microbubbles used as ultrasound contrast agents. Hence, the present invention allows the use of ultrasound with milder, clinically acceptable parameters. The microbubbles according to the invention thus can be easily visualized, concentrated, and destroyed, thereby releasing their therapeutic payload at the site of interest.

## SUMMARY OF THE INVENTION

In a first aspect, the present invention relates to a pharmaceutical composition comprising soft, gas-filled particles having an envelope monolayer comprising one or more surfactants and a pharmaceutically active compound, the envelope monolayer having a negative overall net charge, wherein the pharmaceutically active compound has a positive overall net charge and is directly bound to one or more surfactants by both electrostatic and hydrophobic interactions.

In preferred embodiments of the invention, the pharmaceutically active compound is an anthracycline having a positive overall net charge at a pH of between 4 and 8.

Preferably, the pharmaceutically active compound is selected from the group consisting of doxorubicin, daunorubicin, epirubicin, idarubicin, esorubicin, aclarubicin, and mitoxanthrone. More preferably, the pharmaceutically active compound is doxorubicin.

In further preferred embodiments the surfactant is an amphiphilic compound having one or more saturated alkyl chains with a length of between 10 and 20 carbon atoms, or fluorinated analogues thereof or a combination of said alkyl chains.

In further preferred embodiments the surfactant is an anionic surfactant being a gemini-surfactant or comprising a head group selected from the group consisting of phosphatidylserine (PS), phosphatidylglycerol (PG), phosphatidic acid (PA) and phosphatidylinositol (PI), or a zwitterionic surfactant or non-ionic surfactant, wherein the zwitterionic surfactant comprises a head group selected from phosphatidylcholine (PC) and phosphatidylethanolamine (PE), and wherein the non-ionic surfactant is selected from the group consisting of polysorbate such as Tween®, sorbitan fatty acid ester such as Span® and polyalkylen glycol ether such as Brij®.

In preferred embodiments of the invention at least one surfactant is a phospholipid.

In further preferred embodiments the total surfactant concentration is between 1 mM and 10 mM, preferably between 3 mM and 5 mM.

Also preferably, the molar concentration of anionic surfactants is of between 10 and 50%, preferably of between 20 and 30% based on the total molar surfactant concentration.

In further specific embodiments, the envelope monolayer comprises at least one anionic phospholipid and at least one zwitterionic or non-ionic phospholipid. Preferably, the envelope monolayer comprises phosphatidylcholine and phosphatidylglycerol.

More preferably the envelope monolayer comprises at least one phospholipid comprising a PEG moiety in addition to the at least one anionic and the at least one zwitterionic phospholipid.

Also preferably, the envelope monolayer comprises at least one phospholipid comprising a PEG moiety having a molecular weight from 1,000 to 20,000 Da, more preferably from 2,000 to 10,000 Da.

In further specific embodiments, the molar concentration of the anionic phospholipid is between 10 and 50%, preferably between 20 and 30% based on the total phospholipid content.

In preferred embodiments of the invention, the molar concentration of the pharmaceutically active compound is 10 to 120%, preferably 50 to 110% and more preferably 80 to 100% based on the concentration of the anionic phospholipid.

In further preferred embodiments, the soft, gas-filled particles have a diameter of between 0.5 and 5 $\mu$m, preferably of between 0.8 and 3 $\mu$m.

In a second aspect, the present invention relates to a method of producing the pharmaceutical composition comprising the steps of
(i) providing a pharmaceutically active compound-loaded liposomal preparation comprising the steps of

a) dissolving the one or more surfactants in a polyvalent alcohol,
b) dissolving the pharmaceutically active compound in an aqueous solution, and
c) admixing the compositions of a) and b) to form liposomes comprising large multilamellar vessels (LMV), and
d) homogenizing the LMV obtained in step c) to produce small unilamellar vessels (SUV), and

(ii) subsequent formation of gas-filled particles by adding a pharmaceutically acceptable gas to the pharmaceutical active compound-loaded liposomal preparation.

In further embodiments the produced liposomes have a hydrodynamic diameter of between 100 and 2000 nm, preferably of between 200 and 1000 nm and more preferably of between 400 and 600 nm as determined by dynamic light scattering.

Also preferably, at least 80% of the gas-filled particles have a diameter of between 0.5 and 5 $\mu$m, preferably of between 0.8 and 3 $\mu$m.

In a further aspect, the present invention relates to the use of the pharmaceutical composition comprising soft, gas-filled particles in the treatment of a tumor, wherein the pharmaceutical composition is administered to a mammal in a therapeutically effective amount, and wherein the tumor is selected from the group consisting of solid tumors such as tumors of the brain, liver, kidneys, pancreas, pituitary, colon, breast, lung, ovary cervix, prostate, testicle, oesophagus, stomach, head or neck, bone, or central nervous system.

In preferred embodiments, the tumor is visualized by ultrasound backscattering echo signal produced by the gas-filled particles, wherein the pharmaceutical active agent is released at the site of tumor through destruction of said gas-filled particles by means of diagnostic ultrasound.

## BRIEF DESCRIPTION OF THE DRAWINGS

**Figure 1:**
Effect of agitation time [s] on doxorubicin-content in the microbubble fraction [% of the input amount]. The highest amount of doxorubicin (about 90%) was measured at an agitation time of up to 20 s. Elongated agitation leads to a decrease of doxorubicin in the microbubble fraction.

**Figure 2:**
Effect of cap space volume (filling volume) [% of the vial volume] on microbubble yield [MB count per vial]. The highest yield was achieved at a filling volume of 80%.

**Figure 3**
Anti-proliferative efficacy of doxorubicin-loaded microbubbles according to the invention tested in human carcinoma tissue cultures upon application of ultrasound. An aqueous solution (lane 1), doxorubicin-loaded liposomes (lane 2), unloaded liposomes (lane 3), doxorubicin-loaded microbubbles (lane 4), unloaded microbubbles (lane 5), and a doxorubicin-containing aqueous solution are compared in the experiment. The cell viability [% of untreated cells] in the presence (US (+)) and absence of applied ultrasound (US (-)) was measured. The value marked by the asterisk demonstrates the effect of applied ultrasound on cell viability. The cell viability strongly decreases to less than 20%.

**Figure 4:**
Kinetics of ultrasound-mediated destruction of doxorubicin-loaded microbubbles according to the invention are compared with doxorubicin-loaded triacetin AALs, a marketed ultrasound contrast agent (SonoVue®, Bracco Diagnostics, USA) and a blank treatment (without sonication) in an *in vitro* model (Tinkov et al. 2008). A comparative decrease of microbubble concentration [ml$^{-1}$] was observed for both doxorubicin-loaded microbubbles and Sono-Vue®. The *in vitro*-destructibility of doxorubicin-loaded triacetin AALs was considerably decreased.

**Figure 5:**
Effect of ultrasound treatment on the accumulation of doxorubicin in tumor tissues in an in vivo-model (rat-model) as described in Example 6. Ten test animals (rats) have been subcutaneously implanted pancreas xenograft tumors. Each animal was implanted two tumors, each on one side of the back. One tumor was treated with ultrasound (US (+)) while the other tumor remained untreated (US (-)). After treatment, the test-animals were sacrificed and tissues of tumors and organs were collected. The amount of doxorubicin (µg/g) was measured using C16-RP-HPLC. The measured amount of doxorubicin in ultrasound-treated tumors (US (+)) was a magnitude higher (ten-fold higher) than in untreated tumors (US (-)).

## DETAILED DESCRIPTION OF THE INVENTION

The task of the present invention is to find a simple and efficient way for the targeted delivery of pharmaceutically active agent for use in tumor therapy. The present invention provides a novel acoustically active drug delivery system which is based on a pharmaceutical composition comprising soft, gas-filled particles (microbubbles). It is a further objective to circumvent the disadvantages of conventional microbubble formulations known from the art.

Hence it is an object of this invention to provide a pharmaceutical composition comprising microbubbles loaded with a pharmaceutically active compound that can be easily administered. Another object of the invention is to provide a stable and pharmaceutically acceptable drug carrier suitable for targeted delivery to specific regions of the animal or human body. This requires that the formulation has high drug binding and delivering capacity. It is therefore an objective of the invention to provide a formulation comprising pharmaceutically active compound-loaded microbubbles having advantageous acoustic properties which facilitates the clinical application of mild, pharmaceutically acceptable ultrasound. In particular, the desired acoustic properties should allow for enhanced visualization of the administered microbubbles, localization of the perfused tumor or tissue which is to be targeted and the controlled release at a selected site. Therefore, it is a major objective of the invention to provide an acoustically active drug delivery system comprising microbubbles having higher echogenicity and destructibility. Another object for the invention is to provide an easy to use two-step method for the manufacture of such a pharmaceutical composition. It is further an object to provide a method which mainly uses pharmaceutically acceptable ingredients. Further, the present invention provides a composition that can be prepared as a sterile product and can thus immediately be directly applied as a therapeutic agent by injection. Finally, it is an object of the invention to provide a novel pharmaceutical composition comprising acoustically active particles for use in tumor therapy.

The present invention has solved these problems by making use of the properties of both the pharmaceutically active agent and the composition of the microbubbles. Surprisingly, it was discovered that an advantageous microbubble-containing composition can be formulated such that the pharmaceutically active compound is directly bound to the envelope monolayer of the microbubbles. The binding is based on both electrostatic and hydrophobic interaction between the microbubble shell and the pharmaceutically active compound to be delivered.

The present invention is based on the unexpected finding that the pharmaceutical composition of the present invention comprising soft, gas-filled particles having an envelope monolayer comprising one or more surfactants and a pharmaceutically active compound directly bound to one or more surfactants is ideally suited for the targeted delivery of a therapeutic compound (i.e. antineoplastic drugs, pharmaceuticals, proteins, peptides, antibiotics or oligonucleotides) to specific sites in the human or animal body. The inventive composition is thus particularly suited for tumor therapy. In comparison to compositions known in the art, the inventive composition not only has a sufficiently high stability and drug binding capacity but also exhibits excellent acoustic properties which are essentially required for US mediated monitoring of the drug-loaded microbubbles and the controlled release of the delivered drug. In particular, it was unexpected that microbubbles can be effectively loaded with a large amount of a pharmaceutically active compound while at the same time maintaining or even improving the acoustic properties of the resulting microbubbles. The echogenicity and destructibility of drug-loaded microbubbles according to the invention was unexpectedly found to be enhanced compared to unloaded microbubbles known in the art commonly used as ultrasound contrast agents. Another major advantage of the inventive composition is that it essentially can function with only a minimal amount of pharmaceutically acceptable ingredients.

In order to achieve a high drug binding it is therefore not necessary to dissolve the drug to be carried in an additional oil-layer as suggested in US 2002/0159952. In contrast to the prior art, in the present invention the drug is bound directly to the envelope monolayer via electrostatic and hydrophobic interactions. The invention is clearly distinguishable from the concept of acoustic active liposperes. It is further not necessary to transport the pharmaceutical compound indirectly *via* additional nano-sized vesicle types such as liposomes or micelles as described in WO 2006/127953, WO 99/39738 and US 2007/0081946. The invention is therefore clearly distinguishable from complex compositions having multi-modal and multi-sized vesicle populations. In contrast to the compositions known in the art, the present invention thus provides a pharmaceutical composition that is essentially composed of a mono-modal (uniform) population of nanosized soft, gas-filled particles having a simple, definite, and well characterized structure with consistant, reliable, and reproducible properties.

Another advantage of the invention is that no chemical modification of the pharmaceutically active compound is required for its encapsulation or for its binding to other nanosized-carriers.

The composition according to the invention is further **characterized in that** it is

(1) biocompatible (i.e. substantially non-toxic) and pharmaceutically acceptable,

(2) non allergenic, (3) of non-animal origin (e.g. serum albumin or other proteinaceous substances) and (4) has excellent stability of the drug during manufacturing and release.

Some of the used terms will hereinafter be defined in greater detail below:
Where the term "comprising" is used in the present description and claim, it does not exclude other elements or steps. For the purposes of the present invention, the term "consisting of" is considered to be a preferred embodiment of the term "comprising". If hereinafter a group is defined as comprising at least a certain number of embodiments, this is also to be understood as disclosing a group which preferably consist only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of $\pm$ 10%, preferably 5%.

As used herein, "preparation", "formulation" and "composition" may be used interchangeably herein, and refer to a combination of two or more elements, or substances.

A "solvent" is a liquid which can dissolve gases, other liquids or solid materials without chemical reactions between dissolved matter and dissolving liquid taking place.

As used herein, the terms "subject" or "patient" may be used interchangeably to refer to a mammal that may benefit from the administration of a composition or method as recited herein. Most often the subject or patient will be a human or other mammal such as for example horses, dogs or cats.

As used herein, "administration" and "administering" refer to the manner in which an active agent or composition containing such is presented to a subject.

Concentrations, amounts, solubilities, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "about 1 to about 5" should be interpreted as including not only the explicitly recited values of about 1 to about 5, but also include individual values and sub-ranges within the indicated range. Thus, included in this numerical range are individual values such as 2-3, and 4 sub-ranges such as from1-3, from 2-4 and from 3-5, etc. This same principle applies to ranges reciting only one numerical value. Furthermore, such an interpretation should apply regardless of the breadth of the range or the characteristic being described.

Within the context of the present invention, "mean size" or "mean particle size" defines the median microbubble size, i.e., the microbubble diameter where 50% of the microbubbles are smaller and 50% of the microbubbles are larger than the stated value. Usually, this corresponds to the maximum of a Gaussian size distribution.

The present invention will hereinafter be described with respect to particular embodiments and with reference to certain drawings but the invention is not limited thereto, but only by the claims.

In a first aspect, the present invention relates to a pharmaceutical composition comprising soft, gas-filled particles having an envelope monolayer comprising one or more surfactants and a pharmaceutically active compound, the envelope monolayer having a negative overall net charge, wherein the pharmaceutically active compound has a positive overall net charge and is directly bound to one or more surfactants by both electrostatic and hydrophobic interactions.

As used herein, the terms "gas-filled particles" and "microbubbles" can be used interchangeably and designate air or gas-filled acoustically active particles dispersed in an aqueous medium which are stabilized by an envelope comprising an amphiphilic compound, which is disposed at the gas to liquid interface. Microbubbles can have diameters of between 0.3 $\mu$m and 20 $\mu$m The "envelope", also termed "shell", of microbubbles around a core of gas is mostly evanescent and generally consists of a thin interface layer with a thickness of a few nanometers. Generally, the thickness can be less than 5 nm, typically of about 2-3 nm, often the envelope is a substantially monomolecular layer. Generally, the envelope can be a layer consisting of surfactants, a proteinaceous material, or any other suitable polymeric material.

The term "soft, gas-filled particles" as used herein, particularly designates microbubbles having a "soft" envelope. This is the case when the envelope mainly consists of one or more surfactants. Accordingly, the envelope monolayers of the soft, gas-filled particles of the present invention comprise one or more surfactants.

The term "acoustically active" or "echogenic" as used herein can be used interchangeably to designate the ability of gas-filled particles to create an echo, i.e. to return a signal in the ultrasound field. When gas bubbles are caught in an ultrasonic frequency field, they compress, oscillate, and reflect a characteristic echo which generates a strong and unique sonogram in contrast-enhanced ultrasound.

"Surfactants", also known as tensides, are wetting agents that lower the surface tension of a liquid, allowing easier spreading, and lower the interfacial tension between two liquids. Surfactants are usually organic compounds that are amphiphilic, meaning they contain both hydrophobic groups ("tails") and hydrophilic groups (head groups). Surfactants are therefore soluble in both, organic and aqueous solvents, thus being capable of stabilizing mixtures of otherwise immiscible liquids (e.g. water and oil), mixtures of liquids with gases (e.g. gas microbubbles in water) or mixture of liquids with insoluble particles (e.g. metal nano-particles in water). A surfactant can be generally classified by the presence of the formally charged head group. A non-ionic surfactant has a non-charged head group while the head group of an ionic surfactant carries a net charge. Hence, anionic surfactants have negatively charged head groups; cationic surfactants are positively charged. Surfactants containing a head group with two oppositely charged groups are termed zwitterionic.

As used herein, examples for anionic surfactants (based on sulphate, sulfonate or carboxylate anions) are, for instance, sodium dodecyl sulphate (SDS), ammonium lauryl sulphate, and other alkyl sulphate salts, sodium laureth sulphate (also known as sodium lauryl ether sulphate (SLES)), alkyl benzene sulfonate, soaps and fatty acid salts. Useful anionic surfactants preferably can be surfactants based on a phosphate head group such as phosphatidylserine (PS), phosphatidylglycerol (PC), phosphatidic acid (PA), phosphatidylinositol (PI), or gemini-surfactants such as cardiolipin (CL). Examples for zwitterionic (amphoteric) surfactants are dodecyl betaine, dodecyl dimethylamine oxide, cocamidopropyl betaine, coco ampho glycinate. Other useful phosphate-based zwitterionic surfactants are, for instance, phosphatidylcholine (PC) or phosphatidylethanolamine (PE). As used herein, non-ionic surfactants include alkyl poly(ethylene oxide), copolymers of poly(ethylene oxide) and poly(propylene oxide), alkyl polyglucosides, fatty alcohols, cocamide MEA, cocamide DEA and cocamide TEA. Preferred non-ionic surfactants include polysorbates such as Tween®, sorbitan fatty acid esters such as Span®, and polyalkylen glycol ethers such as Brij®.

The shell composition is substantially dependent on the physico-chemical properties of the used drug such as the hydrophilicity/lipophilicity balance, electrostatic charge, and solubility, which may offer efficient binding mediated by electrostatic or hydrophobic interactions and/or hydrogen bonds. The surfactant composition of the monolayer according to the invention is selected such that the resulting overall net charge of the envelope monolayer is negative. This has the advantage that a positively charged pharmaceutically active compound can directly bind to said one or more surfactants having a negative overall charge by both electrostatic and hydrophobic interaction. Accordingly the pharmaceutically active compound according to the invention requires a positive overall net charge.

As used herein, "direct binding" means that the pharmaceutically active compound has an immediate contact to the surfactant stabilized envelope monolayer. This is in contrast to drug delivery systems, where the pharmaceutical active compound is encapsulated in the inner core of additional nano-sized vesicle types such as liposomes or micelles, which are bound to the outer surface of the microbubbles as described in WO 2006/127953, WO 99/39738 and US 2007/0081946. The invention is therefore clearly distinguishable from these complex compositions having multi-modal and multi-sized vesicle populations. The composition according to the invention thus comprises essentially soft, gas filled particles, wherein the pharmaceutically active compound is directly bound to the amphiphilic surfactant molecules by electrostatic and hydrophobic interaction. This implies that the pharmaceutically active compound can be attached to the outer periphery of the microbubbles, incorporated into the envelope monolayer between the surfactant molecules or bound at the gas/surfactant interface. The present invention is clearly distinguishable from drug delivery systems, which are based on acoustically active lipospheres as disclosed in US 2002/0159952. Acoustic active liposkheres are gas-filled microspheres having a shell consisting of a surfactant monolayer, an underlying oil layer, e.g. triacetin oil. In this case, the additional oil-layer is used to dissolve the pharmaceutically active compound thereby providing a possibly higher drug loading. In contrast, the drug delivery system of the present invention achieves high drug binding without the drug being dissolved and to being carried in an additional oil layer.

As used herein, "active agent", "bioactive agent", "pharmaceutically active agent" and "pharmaceutically active compound" may be used interchangeably to refer to an agent, substance or compound that has measurable specified or selected physiologic activity when administered to a subject in a significant of effective amount. It is to be understood that the term "drug" is also included. Examples of drugs useful in the present invention include, but are not limited to antibiotics, proteins, including antibodies, antibody fragments, receptor molecules, receptor binding molecules, glycoproteins and lectins, peptides, saccharides, steroids, steroid analogs, hormones, cofactors, and genetic material including nucleosides, nucleotides and polynucleotides.

Of specific interest for the present invention are anti-neoplastic or chemotherapeutic agents used to treat cancer in tumor therapy. The advantage of the present invention is that a chemotherapeutic agent can be specifically targeted and released directly at the site of a tumor using diagnostic ultrasound. Examples for chemotherapeutic drugs include for example, but are not limited to alkylating agents, including cisplatin, carboplatin, oxaliplatin, mechlorethamine, cyclophosphamide, chlorambucil, anitmetabolites such as azathiopurine, mercaptopurine, plant alklaloids and terpenoids such as vinca alkaloids, podophyllotoxin and taxanes, topoisomerase inhibitors and antitumor antibiotics.

Preferred embodiments of the present invention are based on the chemical class of anthracycline antibiotics. Anthracyclines are a class of drugs derived from streptomyces bacteria. Generally these compounds are commonly used to treat a wide range of cancers, including leukemias, lymphomas, and breast, uterine, ovarian, and lung cancers.

The pharmaceutical active agent may preferably have a substantially hydrophobic portion which allows for the hydrophobic interaction with the surfactant molecules of the envelope monolayer. As mentioned above, the pharmaceutically active compound used preferably possesses a positive overall net charge for the direct binding to one or more anionic surfactants of the envelope monolayer. In preferred embodiments, the pharmaceutically active compound is an anthracycline having a positive overall net charge at a pH of between 4 and 8. Thus, the cationic property of the used compound determines the binding capacity.

In further preferred embodiments the pharmaceutically active compound is selected from the group consisting of doxorubicin, daunorubicin, epirubicin, idarubicin, esorubicin, aclarubicin, and mitoxanthrone. Most preferably, the anthracycline is doxorubicin.

It is to be understood that "anthracyline" could mean the compound in the form of the free base as well as a salt thereof. According to preferred embodiments of the present invention, the hydrochloride salts of the above anthracyclines are used.

"Doxorubicin", also known as "Adriamycin®" (trade name), has the systematic IUPAC designation (8*S*,10*S*)-10-(4- amino- 5- hydroxy- 6- methyl- tetrahydro- 2*H*-pyran- 2- yloxy- 6,8,11- trihydroxy- 8-(2- hydroxyacetyl)- 1- methoxy-7,8,9,10-tetrahydrotetracene-5,12-dione.

"Daunorubicin" or "daunomycin (daunomycin cerubidine)" has the IUPAC name (8S,10S)-8-acetyl-10-[(2S,4S,5S,6S)-4-amino-5-hydroxy-6-methyl-oxan-2-yl]oxy-6,8,11-trihydroxy-1-methoxy-9,10-dihydro-7H-tetracene-5,12-dione. "

"Epirubicin" has the IUPAC name 10-(4-amino-5-hydroxy-6-methyl-oxan-2-yl)oxy-6,8,11-trihydroxy-8-(2-hydroxyacetyl)-1-methoxy-9,10-dihydro-7H-tetracene-5,12-dione.

"Idarubicine" or 4-demethoxydaunorubicin has the IUPAC name (1*S*,3*S*)-3-acetyl-3,5,12-trihydroxy-6,11-dioxo-1,2,3,4,6,11-hexahydrotetracen-1-yl 3-amino-2,3,6-trideoxo-α-L-*lyxo*-hexopyranoside.

"Esorubicin" also known as "4'-deoxydoxorubicin" has the systemic name (7R,9S)-7-(4-amino-6-methyl-oxan-2-yl)oxy-6,9,11-trihydroxy-9-(2-hydroxyacetyl)-4-methoxy-8,10-dihydro-7H-tetracene-5,12-dione

"Aclarubicin" has the IUPAC name Methyl-(1*R*,2*R*,4*S*)-4-[(2*R*,5*S*,6*S*)-4-dimethylamino-5-[(2*S*,4*S*,5*S*,6*S*)-4-hydroxy-6-methyl-5-[(2*R*,6*S*)-6-methyl-5-oxooxan-2-yl]oxyoxan-2-yl]oxy-6-methyloxan-2-yl]oxy -2-ethyl-2,5,7-trihydroxy-6,11-dioxo-3,4-dihydro-1*H*-tetracene-1-carboxylate.

"Mitoxanthrone" also known as "Novantron" or "Dihydroxyanthrachinone" has the IUPAC name 1,4-dihydroxy-5,8-bis[2-(2-hydroxyethylamino)ethylamino] anthracene-9,10-dione.

The hydrophobic chain length of the envelope surfactant should be in the range of from 10 to 20 carbon atoms. Increasing the number of carbon atoms can lead to a decrease in the surface tension and an increase in gas permeation resistance. Further, increasing the carbon chain length also can increase the shell viscosity, resulting in more robust and less echogenous microbubbles. In order to minimize phase-shift separation and to optimize shell stability, the shell generally should consist of surfactant species possibly having similar alkyl chain length having related transition temperatures. Further, optimized shell stability requires that the shell surfactants according to the invention do not comprise unsaturated alkyl chains. In further embodiments of the present invention, the at least one or more surfactants of the envelope monolayer do not comprise any unsaturated alkyl chains.

In specific embodiments of the present invention, the surfactant is an amphiphilic compound having one or more saturated alkyl chains with a length of between 10 and 20 carbon atoms, or fluorinated analogues thereof or a combination of said alkyl chains.

In further preferred embodiments, the surfactants can have saturated alkyl chains with a length of 14 (myristoyl), 16 (palmitoyl) and 18 (caproyl) carbon atoms. More preferably the surfactants have saturated alkyl chains with a length with 16 (palmitoyl) carbon atoms.

In order to confer the desired negative overall net charge to the envelope monolayer of the microbubbles, the envelope should comprise at least one anionic surfactant. In further preferred embodiments, the surfactant is an anionic surfactant being a gemini-surfactant or comprising a head group selected from the group consisting of phosphatidylserine (PS), phosphatidylglycerol (PG), phosphatidic acid (PA) and phosphatidylinositol (PI), or a zwitterionic surfactant or non-ionic surfactant, wherein the zwitterionic surfactant comprises a head group selected from phophatidylcholine (PC) and phosphatidylethanolamine (PE), and wherein the non-ionic surfactant is selected from the group consisting of polysorbate such as Tween®, sorbitan fatty acid ester such as Span® and polyalkylen glycol ether such as Brij®.

According to preferred embodiments of the present invention at least one of the surfactants forming the microbubbles' envelope is a phospholipid, optionally in admixture with any of the other above cited surfactants.

The term "phospholipid" as used herein refers to lipid molecules containing one or more phosphate groups, including those derived from either glycerol (phosphoglycerides, glycerophospholipids) or sphingosine (sphingolipids). They include polar lipids, and certain phospholipids that are of great importance for the structure and function of cell membranes and are the most abundant of membrane lipids.

Hence, with respect to the objects of the present invention, a phospholipid is an amphiphatic substance which generally contains a hydrophobic group comprising a long chain alkyl group and a hydrophilic group comprising a phosphoric acid group in one molecule. Exemplary phospholipids which may be used in the present invention include glycerophospholipids such as phophatidylcholine (=lecithin), phosphatidylglycerol, phosphatidic acid, phosphatidylethanolamine, phosphatidylserine, and phosphatidylinositol; sphingophospholipids such as sphingomyelin (Sphingomyelin); natural or synthetic diphosphatidyl (or gemini) phospholipids such as cardiolipin, and derivatives thereof.

Examples of preferred phospholipids are dilauroyl-phosphatidylcholine (DLPC), dimyristoyl-phosphatidylcholine (DMPC), dipalmitoyl-phophatidylcholine (DPPC), diarachidoyl-phosphatidylcholine (DAPC), distearoyl- phosphatidylcholine (DSPC), dilauroyl-phosphatidylglycerol (DLPG), diarachidoyl-phosphatidylglycerol (DAPG), dimyristoyl-phosphatidylglycerol (DMPG), distearoyl-phosphatidylglycerol (DSPG), dipalmitoyl-phosphatidylglycerol (DPPG), dimyristoyl phosphatidic acid (DMPA), distearoyl phosphatidic acid (DSPA), dipalmitoyl phosphatidic acid (DPPA), diarachidoyl phosphatidic acid (DAPA), dipalmitoyl-phosphatidylethanolamine (DPPE), distearoyl-phosphatidylethanolamine (DSPE), diarachidoyl-phosphatidylethanolamine (DAPE), dimyristoyl-phosphatidylserine (DMPS), diarachidoyl-phosphatidylserine (DAPS), dipalmitoyl-phosphatidylserine (DPPS), distearoyl-phosphatidylserine (DSPS), dipalmitoyl-sphingomyelin (DPSP), and distearoyl-sphingomyelin (DSSP).

According to preferred embodiments of the present invention, the phospholipids of the envelope monolayer do not comprise any unsaturated alkyl chains. Preferably, the phospholipids have saturated alkyl chains with a length of 14 to 18 carbon atoms, more preferably 16 to 18, even more preferably 16 carbon atoms. Most preferably the envelope monolayer comprises a blend of phospholipids, the blend comprising one or more phospholipids selected from the group consisting of DPPG, DPPC, DPPE, and PEGylated derivatives thereof.

Besides the shell composition, also concentration of the shell ingredients plays a crucial role for the favoured properties of the microbubbles. In particular, the total surfactant concentration has influence on microbubble formulation property (e.g. size, yield, encapsulation, etc.). In preferred embodiments, the total surfactant concentration is between 1 mM and 10 mM, preferably between 3 and 5 mM, more preferably 3 mM.

The term phospholipids as used herein may also include modified phospholipids, i.e. phospholipids, where the hydrophilic head group is bound to another hydrophilic group. Examples of modified phospholipids are phospholipids, where the head group is linked to polyethylenglycol (PEG). PEGylated phospholipids can sterically stabilize microbubbles and it is assumed that they could delay their blood elimination. According to specific embodiments of the present invention, the PEG moieties can have a molecular weight of between 1,000 and 20,000 Da, more preferably of between 2,000 and 10,000 Da, and most preferably of between 2,000 and 5,000 Da. Useful PEGylated phospholipids are, for example, phosphatidylethanolamines such as DPPE-PEG or DSPE-PEG. In a preferred embodiment DPPE-PEG2000 is used to stabilize the microbubble shell in admixture with one or more surfactants.

The selection of the shell excipients dictates some important microbubble properties such as gas osmotic stability, echogenicity, drug binding capacity and destructibility. It has been surprisingly found that a composition comprising at least two phospholipids is advantageous in order to meet the above criteria. In yet further preferred embodiments, the envelope monolayer comprises at least one anionic phospholipid and at least one zwitterionic or non-ionic phospholipid. Thereby, the anionic phospholipid serves to establish the negative overall net charge of the envelope monolayer. Preferably the envelope monolayer comprises phosphatidylcho line and phosphatidylglycerol.

Preferred phosphatidylglycerols which can be used as the anionic surfactant are, for example, dilauroyl-phosphatidylglycerol (DLPG), diarachidoyl-phosphatidylglycerol (DAPG), dimyristoyl-phosphatidylglycerol (DMPG), distearoyl-phosphatidylglycerol (DSPG), and dipalmitoyl-phosphatidylglycerol (DPPG). Most preferred phosphatidylglycerol is dipalmitoyl-phosphatidylglycerol (DPPG).

Useful phosphatidylcholines are, for example, dilauroyl-phosphatidylcholine (DLPC), dimyristoyl-phosphatidylcholine (DMPC), diarachidoyl-phosphatidylcholine (DAPC), and distearoyl- phosphatidylcholine (DSPC). Most preferred phosphatidylcholine used as the zwitterionic surfactant is dipalmitoyl-phophatidylcholine (DPPC).

In further specific embodiments, the envelope monolayer comprises a blend of DPPG and DPPC.

In other specific embodiments, the envelope monolayer according to the invention comprises mixture of at least three phospholipids. In further specific embodiments the shell composition comprises at least one PEGylated phospholipid in addition to the other phospholipids admixed thereto. In further preferred embodiments, the envelope monolayer comprises at least one anionic phospholipid, at least one zwitterionic or non-ionic phospholipid and at least one PEGylated phospholipid.

Preferred shell compositions according to the invention comprise phosphatidylglycerol, phosphatidylcholine and a PEGylated phosphatidylethanolamine. In further preferred embodiments, a blend of DPPG, DPPC and DPPE-PEG has turned out to be highly advantageous with regard to stability and acoustic properties of the resulting microbubbles. In a further specific embodiment, the PEGylated phospholipid is preferably DPPE-PEG2000.

In further specific embodiments, the molar concentration of the PEGylated phospholipid comprised in the pharmaceutical composition is not more than 15 mol%, preferably not more 5 mol%, more preferably of between 1 and 2 mol% based on the total surfactant concentration.

Other excipients or additives may be included in the formulation according to the invention without necessarily being involved in the formation of the stabilizing envelope and may be used in conventional amounts. Hence, in addition to the above mentioned shell ingredients, the pharmaceutical composition may, for instance, contain oligo- or polyvalent alcohols. Preferred oligo- or polyvalent alcohols are, for example, propylene glycol, glycerol or inositol. More preferred is propylene glycol or glycerol. The formulation can further comprise carbohydrates such as glucose, fructose, lactose, trehalose or sucrose. Most preferred carbohydrate is glucose. Preferred concentration of glucose in the pharmaceutical composition is of between 1 and 20 mass%, more preferred of between 3 and 10 mass%, and most preferred about 5 mass% based on the total weight of the liposomal pre-formulation.

In contrast to several microbubbles known in the art, for example, as described in WO 2005/030171 or WO 2006/127953 the envelope of the inventive microbubbles does not require any additional filmogenic protein or block polymer for its stabilization or drug binding. It has surprisingly been found that drug-loaded microbubbles with enhanced acoustic properties can be produced based on simple compositions comprising one or more surfactants, preferably phospholipids. In particular, semi-synthetic phospholipids are generally preferred because of their non-animal origin and the favourable acoustic properties of the resulting phospholipid mono layers. Hence, the inventive composition involves only a minimal selection of substantially pharmaceutically acceptable excipients, which is a prerequisite for parenteral use in the human or animal body.

The drug-binding efficacy of the pharmaceutically active compound having a substantially positive overall net charge is mainly determined by the concentration of anionic surfactants used in the shell composition. In preferred embodiments, the molar concentration of anionic surfactants is of between 10 and 50%, preferably of between 20 and 30% based on the total molar surfactant concentration.

Further, advantageous stabilizing properties of the microbubbles have been achieved at certain concentrations of one or more PEGylated surfactants. In preferred embodiments, the concentration of PEGylated surfactants is maximal 5%, more preferably between 1 to 2% based on the total molar surfactant concentration.

The amount of therapeutic agent used is not only dictated by the effective therapeutic amount. Because the pharmaceutically active compound according to the invention is directly bound to the surfactant shell, the concentration used in the formulations has a direct influence on the microbubble properties, e.g. stability and echogenicity. Surprisingly, it has turned out that the most favourable microbubble properties can be achieved at certain ratios of drug/anionic phospholipid concentrations. The drug-loading capacity of the microbubbles is thus directly limited by the molar concentration of used anionic phospholipids. The molar concentration of the therapeutic compound should therefore not exceed 120% of the molar concentration of anionic phospholipid.

In preferred embodiments of the invention, the molar concentration of the pharmaceutically active compound is 10 to 120%, preferably 50 to 110% and more preferably 80 to 100% based on the concentration of the anionic phospholipid. Most preferably, the concentration of the pharmaceutically active compound is equimolar to the concentration of anionic phospholipid.

The term "core gas" as used herein, designates the microbubble filler gas. The solubility of these gases in water is expressed as liters of gas by liter of water under standard conditions, divided by the square root of the molecular weight. The solubility does not exceed a value of about 0.0003.

The constitution of the gas core can substantially affect the acoustic echogenicity and destructibility of microbubbles. Generally, any biocompatible gas can be employed as the filling gas. As used herein, the gas may comprise, for example, air, nitrogen, oxygen, carbon dioxide, hydrogen, nitrous oxide, an inert gas such, helium, argon, xenon or krypton, alkanes such as methane, ethane, propane, butane, isobutane or cyclopentane, alkens such as propene, butene or isobutene, halogenated gases, preferably fluorinated or perfluorinated low molecular weight hydrocarbons.

In the field of ultrasound imaging, fluorinated gases containing at least one fluorine atom such as fluorinated hydrocarbons, fluorinated halides such as sulfur hexafluoride ($SF_6$), or perfluorocarbons are considered suitable gases. Perfluorocarbons (PFCs) are fluorocarbon compounds derived from hydrocarbons wherein all hydrogen atoms are replaced by fluorine atoms. PFCs are thus made up of carbon and fluorine atoms only.

As used herein, preferred core gases are, for example, selected from the group of perfluorocarbons such as tetrafluormethane ($CF_4$), hexafluorethane ($C_2F_6$), octafluorpropane ($C_3F_8$), decafluorbutane ($C_4F_{10}$), halogenated halides such as sulfur hexafluoride ($SF_6$), nitrogen or a mixture of the aforementioned gases thereof. More preferably, the gas core comprises octafluorpropane ($C_3F_8$), decafluorbutane ($C_4F_{10}$), sulfur hexafluoride ($SF_6$), nitrogen or a mixture of the aforementioned gases.

The clinical versatility of the drug-loaded microbubble formulations is highly dependent on the properties of resulting microbubbles. Quality control and analysis of the microbubbles are crucial in order to ensure biodistribution and therapeutic action in *vivo*. In particular, the size of microbubbles is an important factor with regard to *in vivo* lung clearance and safety, as well as for the microbubbles acoustic response. The microbubble size distribution varies depending on the formulation and production method. Generally, the resulting microbubble size peaks are rather broad and can comprise bi- or multimodal distribution. The upper size limit for microbubbles should be between 5 and 10 μm in order to allow free passage through the lung vasculature.

The size distribution of microbubbles can generally be measured by methods known in the art such as electrical zone sensing, light blockage, laser diffraction or static light scattering, or light microscopy. According to the invention, the mean size distribution is preferably measured using laser diffraction (static light scattering (SLS)). Laser diffraction utilizes the theories of Mie and Fraunhofer to determine particle size distribution from a light scattering pattern. These depend upon analysis of the "halo" of diffracted light produced when a laser beam passes through a dispersion of particles in air or in a liquid. The angle of diffraction increases as particle size decreases, so that this method is particularly good for measuring sizes below 1 $\mu$m Masses and the root mean square radius or a measure of geometric size can be determined using this technique on a mega Dalton scale. Within the context of the present invention, the particle size of the microbubble formulations was determined using static light scattering (Horiba LA-950, Horiba Instruments, Inc., Kyoto Japan) and a properly established optical model. The optical model was created according to Kinoshita et al. [Kinoshita, T., The method to determine the optimum refractive index parameter in the laser diffraction and scattering method. Adv Powder Technol 2001, 12(4): 2858-62.]. In brief, during laser diffraction measurements the detected intensity of the scattered light (s) is mathematically transformed into particle size distribution and back again into estimated light intensity distribution (s*). The conformity of s and s* as a function of the refractive index can be calculated as the cosine of the value of the angle between them (cosine of cross-angle, cos θ). When the optimum refractive index is selected, the optimum particle size distribution is obtained and then is expected that s* will be completely the same or very close to s and cos θ.

In preferred embodiments said gas-filled particles have a diameter of between 0.5 and 5 $\mu$m, preferably of between 0.8 and 3 $\mu$m to allow for the desired acoustic properties.

The produced drug-loaded microbubbles have most favorable acoustic properties such as echogenicity and destructibility. In further preferred embodiments, the soft, gas-filled particles of the present invention are characterized by a high echogenicity and acoustic destructibility, wherein the echogenicity being not less than about 50%, and wherein the acoustic destructibility being not less than about 50% and not more than about 150% based on the echogenicity of the conventional diagnostic ultrasound contrast agent SonoVue® under the same conditions in an *in vitro* experimental setup.

A method to measure the *in vitro* destructibility of microbubble is described herein (Example 5). The *in vitro* model used for this measurement has been described in detail in Tinkov et al. [Tinkov, S., Bekeredjian, R., Winter, G., Coester, C., Characterization of ultrasound-mediated destruction of drug-loaded microbubbles using an improved in vitro model, Applied Acoustics (2008), doi: 10.1016/j.apacoust.2008.10.007]. In brief, the *in vitro* experimental setup of acoustic destructibility testing uses membrane cell, pressurized and brought to 37 °C in order to imitate human blood pressure and body temperature. The optimized egg-like cell shape of the used membrane cell provides optimal flow condition and has a minimized dead volume. Ultrasound with frequencies of 1and 3 MHz and intensities, varying from 1 to 4 W/cm$^2$ was applied through a silicone membrane window to the cell.

In a second aspect, the present invention relates to a method of producing the pharmaceutical composition comprising the steps of

(i) providing a pharmaceutically active compound-loaded liposomal preparation comprising the steps of

a) dissolving the one or more surfactants in a polyvalent alcohol,
b) dissolving the pharmaceutically active compound in an aqueous solution, and
c) admixing the compositions of a) and b) to form liposomes comprising large multilamellar vessels (LMV), and
d) homogenizing the LMV obtained in step c) to produce small unilamellar vessels (SUV), and

(ii) subsequent formation of gas-filled particles by adding a pharmaceutically acceptable gas to the pharmaceutical active compound-loaded liposomal preparation.

The present invention provides a novel and efficient method for producing the pharmaceutical composition comprising pharmaceutically active compound-loaded microbubbles. The pharmaceutical composition according to the invention can also be produced by means of other useful methods of microbubble production well known in the state of the art.

"Liposomes", also known as lipid vesicles, are completely closed lipid bilayer membranes, which contain an entrapped volume comprising an aqueous medium. The lipid bilayer is often composed of phospholipids such as lecithin and related materials such as glycolipids. The bilayer is composed of two lipid monolayers, each having a hydrophobic portion oriented towards each other with the hydrophilic portion facing outwards towards the aqueous phase.

The term liposome includes substantially spherical aggregation of amphiphilic compounds having one or more concentric layers. Liposomes may be, for example, giant unilamellar vesicles (GUVs), large multilamellar vesicles (LUVs), multilamellar vesicles (MLVs) and small unilamellar vesicles (SUVs). The particle size of liposomes can considerably vary, e.g. ≥1000 nm for GUVs, 100 to 1000 nm for LUVs, 200 to 5000 nm for MLVs and ≤ 100nm for SUVs. SUV liposomes can be formed according to conventional techniques known in the art, e.g. by processing of large multilamellar vessels (LMVs), which can be MLV- and/or LUV- suspensions, by ultrasonication, extrusion or microfluidisation. Liposomes smaller than about 20 nm are physically untenable, while liposomes larger than about 1,000 nm in diameter tend to be unstable and aggregate over time. In many therapeutic applications, and particularly in systemic delivery and tissue and cell targeting, liposome size is a critical parameter of therapeutic effectiveness. The liposome particle size can generally be determined by a variety of methods, e.g. by dynamic light scattering (DLS), well known to the persons of ordinary skill in art.

"Dynamic light scattering" (also known as Photon Correlation Spectroscopy (PCS)) is a technique known in the art, which is generally used to determine the size distribution profile of small particles and solutes. The method is based on the fact that shining a monochromatic light beam, such as a laser, onto a solution with spherical particles in Brownian motion causes a Doppler Shift when the light hits the moving particle, changing the wavelength of the incoming light. Rapid fluctuations in scattering intensity, around a mean value at a certain angle, occur because of particle diffusion and are dependent upon on particle size. The calculated correlation function yields a diffusion coefficient, for a given temperature and viscosity, which can be used to calculate particle size.

In preferred embodiments, anionic liposomes loaded with a pharmaceutically active compound may be produced by any conventional state-of-the-art method, which avoids the presence of significant amounts of organic solvents such as ethanol or acetone in the final formulation. According to preferred embodiments of present invention, the liposomal dispersions are produced in a first step as an intermediate pre-formulation, which may further be used to produce drug-loaded microbubbles. The terms "liposomal pre-formulation", "liposomal formulation", and "liposome composition" can hereinafter be used interchangeably.

A liposomal pre-formulation as used herein can be produced using the following methods:

1) Thin film hydration method: The pharmaceutically active compound can be either blended together with the surfactants, or may be directly included in the aqueous phase prior to the thin-film hydration.

2) A one-step method, where the surfactants are dissolved in an appropriate pharmaceutically acceptable polyvalent alcohol, e.g. propylene glycol or glycerol. The pharmaceutically active compound is dissolved in appropriate aqueous medium. Both components are brought to a temperature, at which the physico-chemical complex between surfactants and pharmaceutical active compound persists in a liquid-crystalline phase state. The polyol-phase is mixed with the aqueous phase using a membrane filter for sizing the liposomes until the temperature decreases below the gel to liquid-crystalline main phase transition temperature of the surfactant-drug complex. Subsequently, the formulation can be degassed using e.g. vacuum or ultrasound.

The term "thin film hydration" as used herein designates a method, where lipids are dried onto a solid support such as the side of a glass container and then dispersed by the addition of an aqueous medium, followed by shaking. When hydration occurs in the system, the lipids swell and peel off of the support in sheets to form multilamellar vesicles (liposomes).

The produced liposomes can be further processed in order to optimize their stability, e.g. by spray-drying, freeze-drying, supercritical-gas drying, or others state-of-the-art methods, or can be directly used to produce microbubbles.

In preferred embodiments, the pharmaceutically active compound-loaded liposomal pre-formulation has a total surfactant (e.g. zwitterionic/non-ionic, anionic, and PEGylated surfactant) concentration of between 1 and 30 mM. Preferably, the total surfactant concentration is between 3 and 10 mM. Most preferred are formulations having a total surfactant concentration of 3 mM.

In further preferred embodiments, the concentration of anionic surfactant comprised in the liposomal pre-formulation is of between 5 and 60 mol%, preferably of between 10 and 60 mol%, more preferably of between 20 and 30 mol% based on the total surfactant concentration.

In further specific embodiments, the molar concentration of the PEGylated surfactant comprised in the liposomal pre-formulation is not more than 15 mol%, preferably not more 5 mol%, more preferably of between 1 and 2 mol% based on the total surfactant concentration.

In further preferred embodiments, the molar concentration of zwitterionic or non-ionic surfactant comprised in the liposomal pre-formulation does not exceed 100 mol% based on the concentration of anionic and PEGylated surfactant.

In yet further preferred embodiments, the molar concentration of the pharmaceutically active compound in the liposomal pre-formulation is 10-120%, preferably 50 to 110% and more preferably 80 to 100% to the concentration of anionic phospholipid.

In further preferred embodiments of the present invention, the liposomal pre-formulation comprises less than 10 mM electrolytes, more preferably less than 5 mM, and even more preferably no electrolytes.

The said liposomal pre-formulation may also contain pharmaceutically acceptable polyvalent alcohols (polyols) as listed above. Preferred polyvalent alcohols are propylene glycol or glycerol. Preferably, the concentration of the polyvalent alcohol is not more than 50 vol%, more preferably not more than 20 vol% based on the total formulation volume.

The liposomal formulation may further comprise pharmaceutically acceptable excipients in order to increase the chemical and/or colloidal stability. Preferred excipients are, e.g. chelating agents such as EDTA, disodium EDTA, desferoxamine, lipoic acid, glutathione, or antioxidants such as ascorbic acid palmitate, alpha-tocopherol, ubiquinol, β-carotene, retinol and hydrophilic and/or lipophilic antioxidants. More preferred are excipients selected from the group consisting of EDTA, desferoxamine, glutathione, ubiquinol, hydrophilic antioxidants, ascorbic acid palmitate, and alpha-tocopherol. Most preferred are excipients selected from the group consisting of EDTA, desferoxamine, glutathione, ubiquinol, and hydrophilic antioxidants.

An important aspect of the method according to the invention is that the quality of the resulting drug-loaded microbubble can be steered by formulation parameters of the intermediate liposomes. In particular, the size distribution of liposomes in the pre-formulation is an important characteristic, which determines microbubble quality. As mentioned above, size distribution of the liposomes comprised in the suspension can be measured using dynamic light scattering (DLS). Preferably, the liposomes of the present invention have a hydrodynamic diameter of between 100 and 2000 nm, more preferably of between 200 and 1000 nm and most preferably of between 400 and 600 nm as determined by dynamic light scattering.

Anionic drug-loaded microbubbles can be generally produced by any known method for microbubble production, including mechanical agitation, spray-drying, homprobe sonication, and other combined methods such as the combination of emulsification and drying steps. The preferred method for microbubble production is based on mechanical agitation, whereby the liposomal pre-formulations as described above is agitated at a temperature below the gel to liquid-crystalline main phase transition temperature of the physico-chemical complex between surfactants and drug. Suitable agitation devices for the "activation" of the said liposomal pre-formulation are, e.g. the CapMix® dental amalgamator (3M ESPE, Neuss, Germany) or the VialMix® (Bristol-Myers Squibb Medical Imaging, Inc., North Billerica, MA). In preferred embodiments, the container comprising the liposomal pre-formulation is activated by agitation on its length axis for 10 s to 40 s, preferably for 20 s.

The produced microbubbles can be controlled using static light scattering (SLS). The microbubble mean size is important for the properties such as echogenicity (backscattering activity), acoustic destructibility, drug-loading capacity and microbubble stability. With regard to clinical practice microbubbles having a mean size greater than 5 $\mu$m are harmful since they may cause embolisms. In preferred embodiments of the present invention, at least 80%, preferably more than 90% of the gas-filled particles have a diameter of between 0.5 and 5 $\mu$m, preferably of between 0.8 and 3 $\mu$m based on the surface-area weighted size distribution.

In a further aspect the present invention relates to a pharmaceutical kit for the production of pharmaceutically active compound-loaded microbubbles comprising

1) the above described pharmaceutically active compound-loaded liposomal pre-formulation or any other pre-formulation thereof,
2) an appropriate biocompatible microbubble core gas as described above,
3) an appropriate container, having a preferred shape and an appropriate volume of the said liposomal pre-formulation and microbubble core gas,
4) a device for mechanical agitation of the said container, and
5) an auxiliary dispensing pin for introducing diluting medium into the said container and withdrawing the microbubble suspension in order to avoid over- and underpressure in the container.

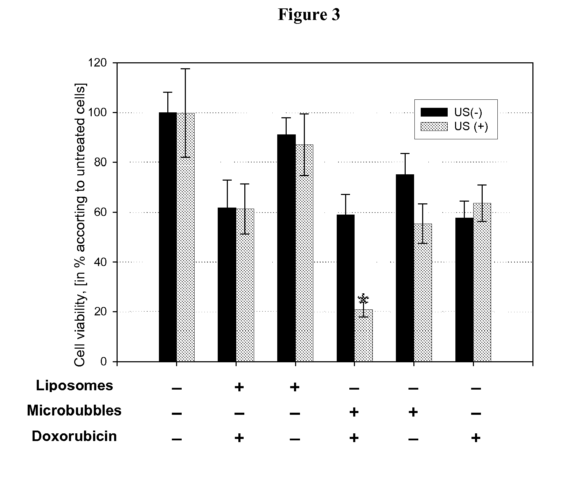

In preferred embodiments, the container used for agitation of drug-loaded liposomal pre-formulation has a volume between 1 and 5 ml. More preferably, the container volume is between 1.5 and 3 ml, most preferably 2 ml (see Figure 3).

In further preferred embodiments the container can be filled with the drug-loaded pre-formulation, wherein the filling volume is between 10 to 50 %, preferably between 10 to 30 %, and most preferably 20 % of the entire container volume.

In a further aspect, the present invention relates to the use of the pharmaceutical composition in the treatment of a tumor, wherein the pharmaceutical composition is administered to a mammal in a therapeutically effective amount, and wherein the tumor is selected from the group consisting of solid tumors such as tumors of the brain, liver, kidneys, pancreas, pituitary, colon, breast, lung, ovary cervix, prostate, testicle, oesophagus, stomach, head or neck, bone, or central nervous system.

In preferred embodiments, the tumor is visualized by ultrasound backscattering echo signal produced by the gas-filled particles, wherein the pharmaceutical active agent is released at the site of tumor through destruction of said gas-filled particles by means of diagnostic ultrasound.

The pharmaceutical composition of the invention is useful to specifically target an anti-neoplastic agent to solid tumors, which can be located in any regions of the human or animal body, and which are accessible for ultrasound having clinically acceptable parameters. Preferably, the tumors should have a developed vasculature and good blood supply. Prior to application, the produced drug-loaded microbubble suspension is diluted with physiological acceptable aqueous solution, e.g. phosphate- buffered saline (PBS) or a glucose solution. The suspension should to be substantially free of atmospheric gases. Preferably, it is saturated with the same gas used as the MB filling gas. The suspension is collected from the vial, while avoiding the occurrence of over- or underpressure in the vial or in the syringe.

The prepared microbubble assembly can be either bolus injected or infused into the vein. The tumor to be targeted has to be located and visualized using the backscattering echo signal, reflected by the administered microbubbles. Accordingly, the time interval of maximum replenishment of the tumor vasculature with microbubbles can be calculated. In clinical studies on rats with subcutaneously implanted xenograft tumors, the time interval was determined a length of about four heart beats. Without being bound to a theory, the length of the time interval can certainly vary depending on vascularisation of the tumor, tumor size and the locus.

"Ultrasound" refers to any mechanistic longitudinal wave with a frequency of above 20 kilohertz (20000 Hz). The production of ultrasound is used in many different fields, typically to penetrate a medium and measure the reflection signature or supply focused energy. The reflection signature can reveal details about the inner structure of the medium. The most well known application of this technique is its use in sonography to produce pictures of fetuses in the human womb. Within the context of the present invention, ultrasound is used to visualize and to disrupt the microbubbles according to the invention due to their deviating impedance relative to blood.

"Mechanical index (MI)" refers to a standard measure of the acoustic output in a diagnostic ultrasound system, defined as the peak rarefactional pressure of an ultrasound longitudinal wave propagating in a uniform medium, divided by the square root of the centre frequency of the transmitted ultrasound pulse. The uniform medium is assumed to have an attenuation of 0.3 dB/cm/MHz (attenuation coefficient divided by ultrasound frequency). The MI determines the interaction of microbubbles with ultrasound, the likelihood of bubble rupture increasing with rising MI.

When the maximal saturation with microbubbles is achieved, a short impulse of ultrasound is directly applied to the tumor. The ultrasound intensity is selected such that it is sufficient to disrupt the microbubbles, thus releasing the delivered drug at the site of the tumor.

Preferably, the non-destructive visualization of the administered microbubbles can be performed by ultrasound with a frequency of about 1.6 MHz and a mechanical index of between 0.3 and 0.8, preferably of between 0.5 and 0.7, and most preferably of about 0.6.

The targeted destruction of microbubbles can be performed using ultrasound having a frequency of about 1.6 MHz and at a mechanical index of not more than 1.9, preferably of between 0.3 and 1.9, more preferably of between 0.8 and 1.9 and even more preferably of between 0.8 and 1.6. Within the context of the invention, the application of the destructive pulses is strictly correlated to the heartbeat. Therefore, the ultrasound pulses are emitted every four systolic contractions.

Tumors, for which the present delivery method is useful, include e.g. solid tumors of the brain, liver, kidneys, pancreas, pituitary, colon, breast, lung, ovary, cervix, prostate, testicle, esophagus, stomach, head or neck, bone, or central nervous system. Ultrasound-mediated targeted destruction of the microbubbles according to the invention can be used to accumulate a pharmaceutical active compound at the site of tumor, which particularly can inhibit or decrease of tumor growth, or induce partial or complete regression of tumors. Ultrasound-mediated targeted destruction of the microbubbles is also useful for preventing the outgrowth of metastases derived from solid tumors.

## EXAMPLES

### Example 1:

### Preparation of doxorubicin hydrochloride loaded microbubbles

At first, Doxorubicin-loaded liposomes are produced according to the thin-film hydration method. Briefly, 17.4 mg dipalmitoyl-phosphatidylcholine (DPPC), 4.5 mg dipalmitoyl-phosphatidylglycerol (DPPG) and 0.8 mg polyethylenglycol-2000-dipalmitoyl-phosphatidylethanolamine (DPPE-PEG2000) were blended in chloroform and the solvent was eliminated at increased temperature under vacuum.

Further, 3.5 mg doxorubicin hydrochloride and 1,000 mg glucose monohydrate were dissolved in 10 ml highly purified electrolyte-free water (Purelab Plus®, USF Elga Ionpure GmbH, Germany). The phospholipid film was hydrated for 60 minutes with the aqueous doxorubicin solution under constant stirring under light- and nitrogen- protection. During the hydration the temperature was maintained above the gel- to liquid-crystalline phase transition temperature of the doxorubicin/phospholipid complex.

The obtained Large Multilamellar Vessels (LMV) were shortly processed with low- frequency horn-type ultrasound homogenizer (Branson Sonifier®, Branson Ultrasonics Corp., USA) to produce Small Unilamellar Vessels (SUV).

The resulting doxorubicin-loaded SUVs were then further analyzed in their size distribution and Zeta potential by dynamic light scattering (Zetasizer Nanoseries , Malvern, Worchestershire, UK). The produced doxorubicin-loaded liposomes had a Zeta potential in electrolyte-free water of between -40 mV and -45 mV. The hydrodynamic diameter of SUV was of between 450 nm and 600 nm and a polydispersity index (PdI) of 0.340 $\pm$ 0.02.

The obtained doxorubicin-loaded liposomes were transferred into 2 ml vials at aliquots of 400 $\mu$l each. The air in the head space was replaced with the microbubble core gas octafluorobutane and the vial was agitated for 20s using a CapMix® dental amalgamator (3M ESPE, Neuss, Germany).

In the resulting doxorubicin-loaded microbubble formulation, the final doxorubicin amount per vial was 140 $\mu$g. The yield of doxorubicin-loaded microbubbles per vial was of between 7 x $10^9$ and 5 x $10^{10}$ particles (measured by light blockage using PAMAS SVSS, PAMAS GmbH, Rutesheim, Germany). The mean size distribution of doxorubicin-loaded microbubbles was of between 800 nm and 2-3 $\mu$m The mean size distribution was determined by static light scattering using Horiba Partica, LA-950 (Horiba Ltd., Kyoto, Japan). The weight ratio of doxorubicin to total phospholipids was 15.3 %. In comparison, the liposomal product Doxil® (Ortho Biotech, USA) has a doxorubicin-to-phospholipid ratio of 13.0 %.

The distribution of the active compound doxorubicin between microbubble fraction, residual liposomal fraction, and free form is depicted in Table 1.

Table 1: Distribution of doxorubicin in the final formulation [% of the overall input amount]

| | |
|---|---|
| Doxorubicin in microbubbles | over 80 % |
| Doxorubicin in liposomes | less than 10 % |
| Free doxorubicin | less than 10 % |

## Example 2:

### Preparation of doxorubicin hydrochloride-loaded microbubbles

In the following, an alternative method for producing doxorubicin hydrochloride-loaded microbubbles is described. In order to produce doxorubicin hydrochloride-loaded liposomes, 17.4 mg dipalmitoyl-phosphatidylcholine (DPPC), 4.5 mg dipalmitoyl-phosphatidylglycerol (DPPG) and 0.8 mg polyethylenglycol-2000-dipalmitoyl-phosphatidylethanolamine (DPPE-PEG2000) were dissolved in 3 ml propylene glycol under heating at 60 °C and vortexing.

The aqueous doxorubicin solution was produced by dissolving 3.5 mg doxorubicin hydrochloride in highly-purified water. The aqueous doxorubicin solution and the phospholipid solution in propylene glycol were heated to 60 °C and filled in syringes.

The syringes were connected with a syringe filter having a pore size of 1 $\mu$m The solutions were extruded until their temperature fell below 30 °C. Liposomes of mean size 0.8 $\pm$ 0.1 $\mu$m and a polydispersity index (PdI) of 0.30 $\pm$ 0.05 were formed.

Upon mechanical agitation at 4.500 oscillations/min for 20 s, doxorubicin-loaded microbubbles were produced having a mean size 0.95 $\mu$m $\pm$ 0.05 $\mu$m and a concentration of 5.4 x $10^9$ particles/ml. About 95 % $\pm$ 1.2 % of the total input amount of doxorubicin hydrochloride was incorporated into the produced microbubbles.

### Example 3:

### Preparation of acoustically active lipospheres (AALs) loaded with doxorubicin

In the following, a method for the production of acoustically active lipospheres- loaded with doxorubicin base is described. Doxorubicin hydrochloride (50 mg) was dissolved in 30 ml water-free methanol and titrated with solution of sodium hydroxide in methanol (5 mg/ml) in stoichiometric proportions in order to obtain doxorubicin base. In addition, a solution of 17.4 mg DPPC, 4.5 mg DPPG, and 0.8 mg DPPE-PEG2000 in 30 ml chloroform was produced. 10 ml triacetin and the phospholipid solution in chloroform were added to the methanolic solution of doxorubicin base.

The mixture was stirred until a homogeneous solution was produced. The organic solvents were eliminated by rotation for two hours under vacuum at 60 °C. Thereby, a clear solution of doxorubicin in triacetin was obtained. The obtained solution of doxorubicin and phospholipids in triacetin was contacted with highly-purified water and stirred for one hour at 60 °C under nitrogen and light protection. The obtained coarse dispersion was then subjected to high-pressure homogenization, resulting in a stable microemulsion. Upon mechanical agitation of this microemulsion acoustically active lipospheres were produced having doxorubicin in the inner triacetin oil layer. The main size of the produced lipospheres was 2.3 $\mu$m $\pm$ 0.3 $\mu$m. The particle yield was determined as being 1.8 x $10^8$ AALs/ml. The encapsulation efficacy of doxorubicin-loaded AALs was determined to be 93% $\pm$ 2% encapsulated doxorubicin, 7% $\pm$ 0.5% doxorubicin in residual emulsion droplets, and 3% $\pm$ 1.3% as free doxorubicin.

The produced AALs were used as a comparative example and were tested in the in vitro destructibility experiment (see Example 5). The acoustic destructibility of doxorubicin-loaded AALs was measured as being approximately 4.5-fold lower than the destructibility of the inventive doxorubicin-loaded soft-shell microbubbles. In view of this, the use of doxorubicin-loaded AALs for ultrasound mediated release of the delivered drug at the target site is therefore considerably restricted. The comparison of these to vesicle types clearly shows the advantageous of doxorubicin-loaded according to the present invention over AALs.

**Example 4:**

**In vitro anti-proliferative efficacy of doxorubicin-loaded microbubbles**

The anti-proliferative efficacy of doxorubicin-loaded microbubbles according to Example 2 was determined in an *in vitro* experiment using 293/KDR human kidney carcinoma tissue cultures.

First, the cells were cultivated in 10% fetal calf serum activated Dulbecco's Modified Eagle Medium. Prior to the experiment, the cells were brought in suspension by trypsin/EDTA solution. A cell suspension of 200 $\mu$l containing about $6 \times 10^4$ cells was transferred into each well of a 96-well plate (Greiner Bio-One GmbH, Germany) and subsequently admixed with an aqueous solution of either doxorubicin, doxorubicin-loaded liposomes, or doxorubicin-loaded microbubbles (n = 10). The corresponding blank samples were prepared without doxorubicin (see Figure 3). Ultrasound with a frequency of 1 MHz, an intensity of 2 W/cm$^2$, and a duty cycle of 50% was applied directly onto each well using Sonitron 2000 (Rich-Mar Inc., USA). In order to avoid the presence of standing waves, the well plate was under laid with an absorbing gel pad.

After 24 hours of incubation the cell metabolic activity was measured using a cell viability assay. Briefly, 20 $\mu$l of a solution of 5 mg/ml 3-(4,5-Dimethylthiazol-2-yl)-2,5-diphenyltetrazolium bromide was added to each well. The cells were then incubated for another two hours. The overlying culture medium was then aspirated off and the cells were solubilized with 50 $\mu$l DMSO. Purple formazan produced by viable cells could be measured by its UV/Vis absorption using FluostarOmega plate reader (BMG Labtech GmbH, Offenburg, Germany) at a wavelength of 590 nm (measurement) and a wavelength of 630 nm (reference). Cell viability was defined as the ratio of the absorption of treated cells to absorption of untreated cell (in percent). The therapeutic efficacy was defined as the loss of cell viability.

Figure 3 shows that the application of ultrasound alone did not have an effect on cell viability. Unloaded microbubbles combined with the application of ultrasound resulted in reduced cell viability of about 69.02% $\pm$ 5.93%. This can be explained by the presence of the cavitation effects. "Cavitation effects" are mechanic events, resulting from the oscillation and fragmentation of MBs. In particular, these are mainly acoustical shock waves, microjets, and microstream shear forces.

The anti-tumor effect of the free doxorubicin or doxorubicin-loaded liposomes was not affected by the application of ultrasound. In both cases, a reduced cell viability of approximately 62-64% was observed. The same effect was achieved when using doxorubicin-loaded microbubbles without the application of ultrasound. However, when doxorubicin-loaded microbubbles were used in combination with ultrasound the observed cell viability was substantially decreased to 17.87% $\pm$ 1.95%. This corresponds to a 5.71-fold $\pm$ 1.48 reduction of cell viability. Free doxorubicin resulted in only a moderate reduction (1.77-fold $\pm$ 0.46) of cell viability. A similar effect has been achieved with doxorubicin-loaded liposomes (1.68-fold $\pm$ 0.56). Hence, there was no statistically significant difference compared to free doxorubicin. The use of doxorubicin-loaded microbubbles without ultrasound resulted in a decrease of 1.62-fold $\pm$ 0.43.

Although unloaded microbubbles combined with ultrasound have been shown to possess an intrinsic cell inhibition activity (1.49 fold $\pm$ 0.37), it was unexpected that the anti-proliferative effect of doxorubicin loaded microbubbles combined with ultrasound was about 175% greater than the additive inhibiting effect of free doxorubicin and unloaded microbubbles with ultrasound. Hence, the experiment clearly demonstrates the advantages of the doxorubicin-loaded microbubbles according to the invention. In combination with ultrasound, the inventive drug-loaded microbubbles thus revealed a synergistic therapeutic effect of about 150%.

**Example 5:**

**in vitro destructibility of doxorubicin-loaded microbubbles**

Doxorubicin-loaded microbubbles have been produced according to the method as described in Example 1. The pharmaceutical composition was tested with regard to its acoustic destructibility. The acoustic destructibility of the inventive doxorubicin-loaded microbubble formulation was compared in an *in vitro*-model with formulations comprising unloaded microbubbles, acoustically active lipospheres (AALs) having an additional layer of triacetin (see Example 3), as well as with a marketed microbubble containing contrast agent (SonoVue®, Bracco Diagnostics, USA). The *in vitro* model has been described in detail in Tinkov et al. [Tinkov, S., Bekeredjian, R., Winter, G., Coester, C., Characterization of ultrasound-mediated destruction of drug-loaded microbubbles using an improved in vitro model, Applied Acoustics (2008), doi: 10.1016/j.apacoust.2008.10.007]

In brief, the *in vitro* experimental setup of acoustic destructibility testing used membrane cell, pressurized and brought to 37 °C in order to imitate human blood pressure and body temperature. The optimized egg-like cell shape of the used membrane cell provided optimal flow conditions and had a minimized dead volume. Ultrasound with frequencies of 1 and 3 MHz and intensities, varying from 1 to 4 W/cm$^2$ was applied through a silicone membrane window to the cell. Microbubbles size distribution and concentration were measured by light blockage in equal time intervals during the sonication.

The optimized *in vitro* setup demonstrated differences in the ultrasound destructibility of the microbubble formulations used. The fastest decay upon ultrasound exposure was found for SonoVue®. Unloaded and drug-loaded microbubbles according to the invention appeared to be comparably destructible to SonoVue®. AALs were about 4.5-fold more stable, i.e. less destructible, than SonoVue®.

**Table 2: Coefficient of determination $R^2$, decay function parameters (*a* and *b*), and half-life of different microbubble (MB) formulations during sonication at a frequency of 1 MHz and output intensity of 4 W/cm$^2$ (number of experiments n = 5, number of measurements per experiment, m = 3).**

| Microbubble type | $R^2$ | Initial population, *a* | Decay Coeffizient, *b* | Half-life [s] |
|---|---|---|---|---|
| Blank measurement | 0.8220 | $1.048 \times e^5$ | $3.622 \times e^{-4}$ | $1913 \pm 0.9$ |
| Triacetin AALs | 0.9605 | $1.073 \times e^5$ | $3.769 \times e^{-3}$ | $184.02 \pm 3.12$ |
| Unloaded MBs | 0.9363 | $1.030 \times e^5$ | $1.530 \times e^{-2}$ | $60.15 \pm 2.63$ |
| Drug-loaded MBs | 0.9450 | $1.053 \times e^5$ | $1.152 \times e^{-2}$ | $60.18 \pm 1.32$ |
| SonoVue® | 0.9894 | $9.693 \times e^4$ | $1.688 \times e^{-2}$ | $41.05 \pm 1.83$ |

**Example 6:**

**In vivo-echogenicity and acoustic destructibility of doxorubicin-loaded microbubbles**

Acoustic back-scatter efficacy (echogenicity) and destructibility of doxorubicin-loaded microbubbles were tested in myocardium *in vivo* model using rats. The formulation comprising doxorubicin-loaded microbubbles were compared with a commercially available contrast agent formulation (SonoVue®, Bracco Diagnostics, USA SonoVue®).

After the test animals were prepared, microbubble dispersions produced according to Example 2 were infused at a rate of 3 ml/hour. During the infusion, the left heart was visualized by diagnostic ultrasound (Sonos 5500, Philips Medical Systems, USA) having a frequency of 1.3 MHz and a mechanical index (MI) of 0.6. Following the visualization, the left heart was exposed to bursting cycles with the same frequency and a higher MI of 1.6. A sequence of four bursting pulses was delivered every fourth diastolic cardiac cycle. It was triggered by the electrocardiographic R-wave with a delay of 80 ms after its peak.

Immediately prior and after every bursting sequence backscatter images were taken in order to calculate the rate of microbubble destructibility. Backscattered signal intensity was recorded immediately prior to the high-intensity ultrasound sequence ($I_{bs}$) and after it ($I^*_{bs}$). The microbubble *in-vivo* acoustic destructibility A, [%] was calculated according to the following equation:

$$A\% = \left(1 - \frac{I_{bs}}{I^*_{bs}}\right) \times 100$$

**Calculation of the *in vivo*-acoustic destructibility A, [%]:**

A - microbubble *in-vivo* acoustic destructibility, [%]; $I^*_{bs}$- backscatter intensity before the bursting frame, [dB]; $I_{bs}$- backscatter intensity after the bursting frame, [dB].

Surprisingly, it has been found that doxorubicin-loaded microbubbles possessed excellent echogenicity when compared to the commercial standard SonoVue®. It was unexpected that the *in vivo*-acoustic destructibility of the inventive drug-loaded microbubbles was 1.75-fold higher than the destructibility of unloaded SonoVue®- microbubbles.

**Table 3: Opacification intensity, [dB] and acoustic destructibility, [%] of doxorubicin-loaded microbubbles compared to the marketed ultrasound contrast agent SonoVue®.**

|  | Doxorubicin-loaded MBs | SonoVue® |
|---|---|---|
| Opacification intensity, [dB] | 35.10 | 28.24 |
| Destructibility, [%] | 70.01 | 40.74 |

**Example 7:**

***In vivo*-drug delivery efficacy of doxorubicin-loaded microbubbles**

Ten test animals (rats) have been subcutaneously implanted pancreas xenograft tumors. Each animal was implanted two tumors implanted, each on one side of the back. The doxorubicin-loaded microbubble formulation as described in Example 1 was administered to all test animals by infusion through the carotid artery. Each animal was infused one vial of 400 μl microbubbles, corresponding to a dose of 140 μg doxorubicin hydrochloride.

Simultaneously, while one of the tumors of each animal was treated with ultrasound, the tumor on the other half (control) of the back of the test animals remained untreated. Ultrasound with a frequency of 1.3 MHz was applied. The microbubbles were visualized by ultrasound having a mechanical index of 0.6. Every four heartbeats ultrasound pulses with a higher mechanical index of 1.6 was applied in order to disrupt the microbubbles and to release the encapsulated doxorubicin at the site of the tumor.

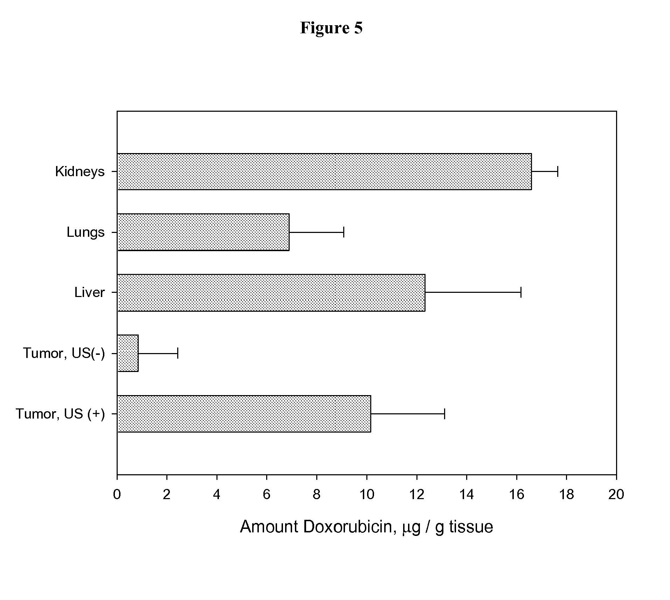

Subsequently, the treated test animals were sacrificed and the tumor tissues were collected along with the liver, lungs, and kidneys. The tissues of tumor and organs were homogenized, and the containing doxorubicin was extracted and quantified using C16-reversed phase-HPLC equipped with UV and fluorescence detectors. Surprisingly, the measured amount of doxorubicin in ultrasound-treated tumors was a magnitude higher (ten-fold higher) than in untreated tumors (Figure 5).

**Claims**

1.  A pharmaceutical composition comprising soft, gas-filled particles having an envelope monolayer comprising one or more surfactants and a pharmaceutically active compound, the envelope monolayer having a negative overall net charge, wherein the pharmaceutically active compound has a positive overall net charge and is directly bound to one or more surfactants by both electrostatic and hydrophobic interactions.

2.  The pharmaceutical composition according to claim 1, wherein the pharmaceutically active compound is an anthracycline having a positive overall net charge at a pH of between 4 and 8.

3.  The pharmaceutical composition according to claim 1 or 2, wherein the pharmaceutically active compound is selected from the group consisting of doxorubicin, daunorubicin, epirubicin, idarubicin, esorubicin, aclarubicin, and mitoxanthrone.

4.  The pharmaceutical composition according to any of claims 1 to 3, wherein the surfactant is an amphiphilic compound having one or more saturated alkyl chains with a length of between 10 and 20 carbon atoms, or fluorinated analogues thereof or a combination of said alkyl chains.

5.  The pharmaceutical composition according to any of claims 1 to 4, wherein at least one surfactant is an anionic surfactant being a gemini-surfactant or comprising a head group selected from the group consisting of phosphatidylserine (PS), phosphatidylglycerol (PG), phosphatidic acid (PA) and phosphatidylinositol (PI), or a zwitterionic surfactant or non-ionic surfactant, wherein the zwitterionic surfactant comprises a head group selected from phosphatidylcholine (PC) and phosphatidylethanolamine (PE), and wherein the non-ionic surfactant is selected from the group consisting of polysorbate, sorbitan fatty acid ester and polyalkylen glycol ether.

6.  The pharmaceutical composition according to any of claims 1 to 5, wherein at least one surfactant is a phospholipid.

7.  The pharmaceutical composition according to any of claims 1 to 5, wherein the total surfactant concentration is between 1 mM and 10 mM, preferably between 3 and 5 mM.

8.  The pharmaceutical composition according to any of claims 1 to 7, wherein the envelope monolayer comprises at least one anionic phospholipid and at least one zwitterionic or non-ionic phospholipid.

9. The pharmaceutical composition according to any of claims 1 to 8, wherein the envelope monolayer comprises phosphatidylcholine and phosphatidylglycerol.

10. The pharmaceutical composition according to any of claims 6 to 9, wherein the phospholipid comprises a PEG moiety having a molecular weight from 1,000 to 20,000 Da, more preferably from 2,000 to 10,000 Da.

11. The pharmaceutical composition according to any of claims 8 to 10, wherein the molar concentration of the anionic phospholipid is between 10 and 50%, preferably between 20 and 30% based on the total phospholipid content.

12. The pharmaceutical composition according to any of claims 8 to 11, wherein the molar concentration of the pharmaceutically active compound is 10 to 120%, preferably 50 to 110% and more preferably 80 to 110% to the concentration of the anionic phospholipid.

13. The pharmaceutical composition according to any of claims 1 to 12, wherein said gas-filled particles have a diameter of between 0.5 and 5 $\mu$m, preferably of between 0.8 and 3 $\mu$m.

14. A method of producing the pharmaceutical composition according to any of claims 1 to 13, comprising the steps of

    (i) providing a pharmaceutically active compound-loaded liposomal preparation comprising the steps of

        a) dissolving the one or more surfactants in a polyvalent alcohol,
        b) dissolving the pharmaceutically active compound in an aqueous solution,
        and
        c) admixing the compositions of a) and b) to form liposomes comprising large multilamellar vessels (LMV), and
        d) homogenizing the LMV obtained in step c) to produce small unilamellar vessels (SUV), and

    (ii) subsequent formation of gas-filled particles by adding a pharmaceutically acceptable gas to the pharmaceutical active compound-loaded liposomal preparation.

15. The method according to claim 14, wherein the liposomes obtained in (i) have a hydrodynamic diameter of between 100 and 2000 nm, preferably of between 200 and 1000 nm and more preferably of between 400 and 600 nm as determined by dynamic light scattering.

16. The method according to claim 14 or 15, wherein at least 80% of the gas-filled particles have a diameter of between 0.5 and 5 $\mu$m, preferably of between 0.8 and 3 $\mu$m.

17. Use of the pharmaceutical composition according to any of claims 1 to 13 in the treatment of a tumor, wherein the pharmaceutical composition is administered to a mammal in a therapeutically effective amount, and wherein the tumor is selected from the group consisting of solid tumors such as tumors of the brain, liver, kidneys, pancreas, pituitary, colon, breast, lung, ovary cervix, prostate, testicle, oesophagus, stomach, head or neck, bone, or central nervous system.

18. The use of claim 17, wherein the tumor is visualized by ultrasound backscattering echo signal produced by the gas-filled particles, wherein the pharmaceutical active agent is released at the site of tumor through destruction of said gas-filled particles by means of diagnostic ultrasound.

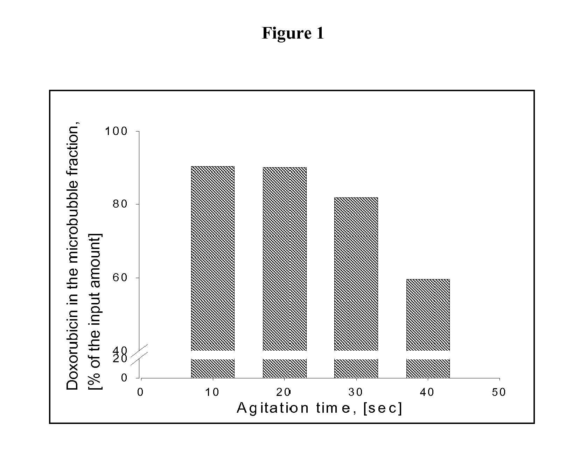

**Figure 1**

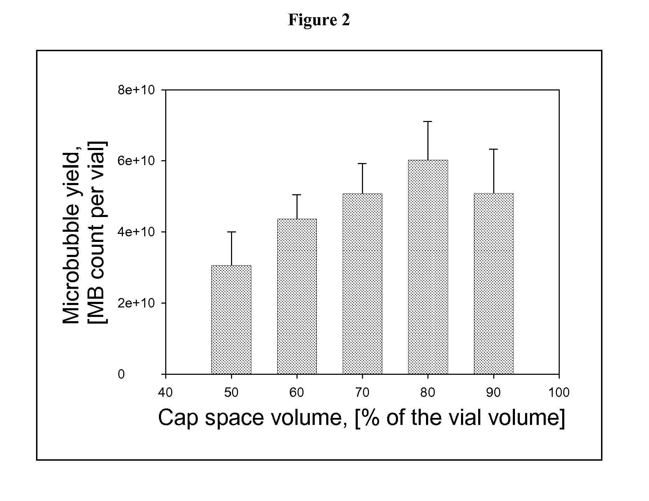

Figure 2

## Figure 3

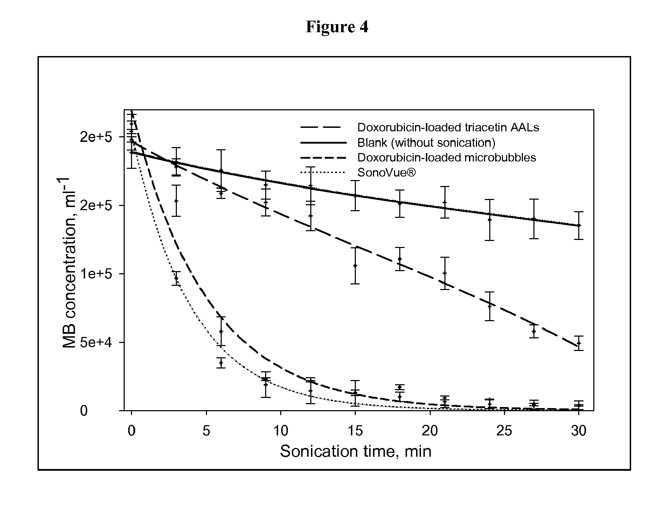

**Figure 4**

**Figure 5**

**Europäisches Patentamt**

**European Patent Office**

**Office européen des brevets**

# PARTIAL EUROPEAN SEARCH REPORT

**Application Number**

which under Rule 63 of the European Patent Convention EP 09 16 0913
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/088176 A1 (UNGER EVAN C [US]) 8 May 2003 (2003-05-08) * paragraphs [0089], [0099], [0100]; example 7 * | 1-18 | INV. A61K9/127 A61K49/22 A61K31/704 A61K9/133 |
| X | US 2003/044354 A1 (CARPENTER ALAN P [US] ET AL) 6 March 2003 (2003-03-06) * paragraph [0065]; claim 28; examples 3,6 * | 1-18 | |
| X | WO 94/28874 A (UNGER EVAN C [US]; FRITZ THOMAS A [US]; MATSUNAGA TERRY [US]; RAMASWAM) 22 December 1994 (1994-12-22) * page 33, last paragraph * * page 38, line 20 - line 21 * * page 43, line 2 - line 3 * * claims 13,14; example 2 * | 1-18 | |
| A | WO 2008/120998 A (EPITARGET AS [NO]; FOSSHEIM SIGRID L [NO]; LAUTEN CECILIA LEAL [NO]; J) 9 October 2008 (2008-10-09) * page 7, paragraph 1 * * page 8, paragraph 2 * * page 8, last paragraph * * page 9, paragraph 1-3 * | 1-18 | TECHNICAL FIELDS SEARCHED (IPC) A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 22 September 2009 | Giménez Miralles, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches
Patentamt

European
Patent Office

Office européen
des brevets

**INCOMPLETE SEARCH
SHEET C**

Application Number

EP 09 16 0913

Although claims 17 and 18 are directed to a method of treatment of the human/animal body (Article 53(c) EPC), the search has been carried out and based on the alleged effects of the compound/composition.

-----

Claim(s) not searched:
        17,18

Reason for the limitation of the search (non-patentable invention(s)):

Article 53 (c) EPC - Method for treatment of the human or animal body by therapy

## ANNEX TO THE EUROPEAN SEARCH REPORT
## ON EUROPEAN PATENT APPLICATION NO.

EP 09 16 0913

This annex lists the patent family members relating to the patent documents cited in the above-mentioned European search report.
The members are as contained in the European Patent Office EDP file on
The European Patent Office is in no way liable for these particulars which are merely given for the purpose of information.

22-09-2009

| Patent document cited in search report | Publication date | Patent family member(s) | Publication date |
|---|---|---|---|
| US 2003088176 A1 | 08-05-2003 | NONE | |
| US 2003044354 A1 | 06-03-2003 | NONE | |
| WO 9428874 A | 22-12-1994 | AU 696056 B2 | 27-08-1998 |
| | | AU 6953794 A | 03-01-1995 |
| | | CA 2164846 A1 | 22-12-1994 |
| | | EP 0802788 A1 | 29-10-1997 |
| | | JP 8511523 T | 03-12-1996 |
| WO 2008120998 A | 09-10-2008 | US 2009098212 A1 | 16-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 8809168 A
- US 4898735 A
- US 4797285 A
- WO 2007137326 A
- WO 2005030171 A
- WO 2006127953 A
- US 20020159952 A
- WO 199939738 A
- US 20070081946 A
- WO 9939738 A

### Non-patent literature cited in the description

- **Rapoport, N.** Controlled Drug Delivery to Drug Sensitive and Multidrug Resistant Cells: Effects of Pluronic Micelles and Ultrasound in: Advances in Controlled Drug Delivery. ACS Symposium Book Series, 2003, 85-101
- **Perez-Soler, R. ; Priebe, W.** Liposomal formulation and antitumor activity of 14-O-palmitoyl-hydroxyrubicin. *Cancer Chemother Pharmacol,* 1992, vol. 30, 267-271
- **Siwak, D. ; Tary, A.M. ; Lopes-Berestein, G.** The potential of drug-carrying immunoliposomes as anticancer agents. *Clin Canc Res,* 2002, vol. 8, 955-956
- **Amselem, S. et al.** Optimization and upscaling of doxorubicin-containing liposomes for clinical use. *J Pharm Scie,* 1990, vol. 79 (12), 1045-1052
- **Burstein, O. ; Wheatley, M.A.** Drug loaded contrast agents for cancer tumors: A combination of imaging and therapy. Biomedical Technology Showcase, School of Biomedical Engineering. *Science& health Systems,* 2006
- **Kinoshita, T.** The method to determine the optimum refractive index parameter in the laser diffraction and scattering method. *Adv Powder Technol,* 2001, vol. 12 (4), 2858-62
- **Tinkov, S. ; Bekeredjian, R. ; Winter, G. ; Coester, C.** Characterization of ultrasound-mediated destruction of drug-loaded microbubbles using an improved in vitro model. *Applied Acoustics,* 2008